# FASCICULE DE BREVET EUROPEEN

(11) **EP 1 701 946 B1**
(45) Date de publication et mention de la délivrance du brevet: **19.12.2007**
(21) Numéro de dépôt: 04816424.8
(22) Date de dépôt: 17.12.2004
(51) Int. Cl.: C07D 295/185, C07D 311/04, C07D 317/58, C07D 307/81, C07D 495/04

(54) **DERIVES DE 1-PIPERAZINE- ET 1-HOMOPIPERAZINE-CARBOXYLATES, LEUR PREPARATION ET LEUR APPLICATION EN TANT QU'INHIBITEURS DE L'ENZYME FAAH**
DERIVATE VON 1-PIPERAZIN- UND 1-HOMOPIPERAZINCARBOXYLATEN, VERFAHREN ZU DEREN HERSTELLUNG UND DEREN VERWENDUNG ALS INHIBITOREN DES FAAH-ENZYMS
DERIVATIVES OF 1-PIPERAZINE- AND 1-HOMOPIPERAZINE-CARBOXYLATES, PREPARATION METHOD THEREOF AND USE OF SAME AS INHIBITORS OF THE FAAH ENZYME

(30) Priorité: 23.12.2003 FR 0315248
(43) Date de publication de la demande: 20.09.2006
(73) Titulaire: Sanofi-Aventis, 75013 Paris (FR)
(72) Inventeur: ABOUABDELLAH, Ahmed, F- 91270 Vigneux-Sur-Seine (FR); ALMARIO GARCIA, Antonio, F-92290 Chatenay Malabry (FR); HOORNAERT, Christian, F-92160 Antony (FR); LI, Adrien, Tak, F-92260 Fontenay aux Roses (FR)
(74) Mandataire: Morel-Pécheux, Muriel
(86) Numéro de dépôt international: PCT/FR2004/003289
(87) Numéro de publication internationale: WO 2005/070910

(56) Documents cités:
- EP-A- 0 548 798
- WO-A-01/72728
- WO-A-97/14689
- WO-A-03/097573
- WO-A-20/04099176

## Description

L'invention a pour objet des dérivés de 1-pipérazine- et 1-homopipérazine-carboxylates, leur préparation et leur application en thérapeutique.

Des dérivés de pipéridine et pipérazine utiles pour le traitement de la douleur, de la migraine ou des maladies neurodégénératives sont décrits dans les demandes de brevet WO03/097573 et WO97/14689.

La demande de brevet WO01/72728 décrits des dérivés de pipérazine utiles dans le traitement des maladies inflammatoires .

La demande de brevet EP-A-0 548 798 décrit des dérivés de 4-phényl-pipéridine et pipérazine comme agents antiviraux.

Des inhibiteurs de FAAH comportant une structure centrale amine cyclique substituée par un groupement comportant une fonction carbamate sont décrits dans WO2004/099176.

Les composés de l'invention répondent à la formule générale (I) 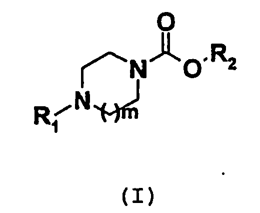 dans laquelle
m représente un nombre entier égal à 1 ou 2 ;
R₁ représente un groupe choisi parmi notamment un phényle, pyridinyle, pyridazinyle, pyrimidinyle, pyrazinyle, triazinyle, oxazolyle, thiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, cinnolinyle, naphthyridinyle, benzofuranyle, dihydrobenzofuranyle, benzothiényle, dihydrobenzothiényle, indolyle, benzoxazolyle, benzisoxazolyle, benzothiazolyle, benzisothiazolyle, benzimidazolyle, indazolyle, pyrrolopyridinyle, furopyridinyle, dihydrofuropyridinyle, thiénopyridinyle, dihydrothiénopyridinyle, imidazopyridinyle, imidazopyrimidinyle, pyrazolopyridinyle, oxazolopyridinyle, isoxazolopyridinyle, thiazolopyridinyle, isothiazolopyridinyle,
ce groupe étant éventuellement substitué par un ou plusieurs groupes R₃ identiques ou différents l'un de l'autre ou par un groupe R₄ ;
R₂ représente un groupe de formule générale CHR₅CONHR₆,
R₃ représente un atome d'halogène ou un groupe hydroxy,cyano, nitro, C₁₋₆-alkyle, C₁₋₆-alcoxy, C₁₋₆-thioalkyle, C₁₋₆-fluoroalkyle, C₁₋₆-fluoroalcoxy, -O-(C₂₋₃-alkylène)-, -O- (C₁₋₃-alkylène)-O-, C₁₋₆-fluorothioalkyle, C₃₋₇-cycloalkyle, C₃₋₇-cycloalkyle-C₁₋₃-alkylène, pipéridinyle, benzyloxy, pipérazinyle, pyrrolidinyle, morpholinyle, phényloxy, NR₇R₈, NHCOR₇, NHSO₂R₇, COR₇, CO₂R₇, CONR₇R₈, SO₂R₇ ou SO₂NR₇R₈, R₄ représente un groupe choisi parmi notamment un phényle, benzofuranyle, naphtyle, pyridinyle, pyrimidinyle, pyridazinyle, pyrazinyle, triazinyle, oxazolyle, thiazolyle, imidazolyle, oxadiazolyle, thiadiazolyle, triazolyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazolpyrimidinyle, benzothiényle, indolyle, benzoxazolyle, benzisoxazolyle, benzothiazolyle, benzisothiazolyle, benzimidazolyle, indazolyle, pyrrolopyridinyle, furopyridinyle, dihydrofuropyridinyle, thiénopyridinyle, dihydrothiénopyridinyle, imidazopyridinyle, imidazopyrimidinyle, pyrazopyridinyle, oxazolopyridinyle, isoxazolopyridinyle, thiazolopyridinyle, isothiazolopyridinyle ;
le ou les groupes R₄ pouvant être substitués par un ou plusieurs groupes R₃ identiques ou différents l'un de l'autre ;
R₅ représente un atome d' hydrogène ou un groupe C₁₋₃-alkyle ; R₆ représente un atome d'hydrogène ou un groupe C₁₋₆-alkyle, C₃₋₇-cycloalkyle ou C₃₋₇-cycloalkyle-C₁₋₃-alkylène ;
R₇ et R₈ représentent, indépendamment l'un de l'autre, un atome d'hydrogène, un groupe C₁₋₃-alkyle ou un groupe phényle.

Parmi les composés de formule générale (I), un premier sous-groupe de composés est constitué des composés pour lesquels :
m représente un nombre entier égal à 1 ou 2 ; et/ou
R₁ représente un groupe choisi parmi notamment un phényle, pyridinyle, pyrimidinyle, pyrazinyle, naphtyle, quinolinyle, isoquinolinyle, benzisoxazolyle, thiénopyridinyle, ce groupe étant éventuellement substitué par un ou plusieurs groupes R₃, plus particulièrement par un ou deux groupes R₃ identiques ou différents l'un de l'autre ; et/ou
R₂ représente un groupe de formule générale CHR₅CONHR₆ ; et/ou
R₃ représente un atome d'halogène, plus particulièrement un chlore, un brome ou un fluor, ou un groupe cyano, C₁₋₆-alkyle, plus particulièrement un méthyle, éthyle, n-propyle, isobutyle, C₁₋₆-alcoxy, plus particulièrement un méthoxy, C₁₋₆-fluoroalkyle, plus particulièrement un CF₃, C₁₋₆-fluoroalcoxy, plus particulièrement un -OCH₂CF₃, -O-(C₂₋₃-alkylène)-, plus particulièrement un -O-(CH₂)₃-, phényloxy ; et/ou
R₅ représente un atome d'hydrogène ; et/ou
R₆ représente un atome d' hydrogène ou un groupe C₁₋₆-alkyle, plus particulièrement un méthyle.

Parmi les composés de formule générale (I), un second sous-groupe de composés est constitué des composés pour lesquels :
m est égal à 1 ; et/ou
R₁ représente un groupe choisi parmi notamment un pyridinyle, pyrimidinyle, pyrazinyle, quinolinyle, isoquinolinyle, ce groupe étant éventuellement substitué par un groupe R₃ ; et/ou
R₂ représente un groupe de formule générale CHR₅CONHR₆; et/ou
R₃ représente un atome d'halogène, plus particulièrement un chlore, ou un groupe C₁₋₆-alkyle, plus particulièrement un méthyle, éthyle, n-propyle, isobutyle, C₁₋₆-alcoxy, plus particulièrement un méthoxy, C₁₋₆-fluoroalkyle, plus particulièrement un CF₃ ; et/ou
R₅ représente un atome d'hydrogène ; et/ou
R₆ représente un atome d'hydrogène ou un groupe C₁₋₆-alkyle, plus particulièrement un méthyle.

Parmi les composés de formule générale (I), un troisième sous-groupe de composés est constitué des composés pour lesquels :
m représente un nombre entier égal à 1 ou 2 ; et/ou
R₁ représente un groupe choisi parmi notamment un phényle, pyridinyle, pyridazinyle, pyrimidinyle, thiadiazolyle, ce groupe étant éventuellement substitué par un groupe R₄; et/ou
R₄ représente un groupe choisi parmi notamment un phényle, benzofuranyle, naphtyle ; le groupe R₄ pouvant être substitué par un ou plusieurs groupes R₃ identiques ou différents l'un de l'autre, plus particulièrement par un ou deux groupes R₃ identiques ou différents l'un de l'autre ; et/ou
R₂ représente un groupe de formule générale CHR₅CONHR₆ ; et/ou
R₃ représente un atome d'halogène, plus particulièrement un chlore, un brome ou un fluor, ou un groupe nitro, C₁₋₆-alkyle, plus particulièrement un méthyle, isopropyle, C₁₋₆-alcoxy, plus particulièrement un méthoxy, éthoxy, C₁₋₆-fluoroalkyle, plus particulièrement un CF₃, C₁₋₆-fluoroalcoxy, plus particulièrement un OCF₃, -O-(C₁₋₃-alkylène)-O-, plus particulièrement un -O-CH₂-O-, benzyloxy ; et/ou
R₅ représente un atome d'hydrogène : et/ou
R₆ représente un atome d'hydrogène ou un groupe C₁₋₆-alkyle, plus particulièrement un méthyle ou un éthyle, ou C₃₋₇-cycloalkyle-C₁₋₃-alkylène, plus particulièrement un cyclopropyl-CH₂-.

Parmi les composés de formule générale (I), un quatrième sous-groupe de composés est constitué des composés pour lesquels :
m est égal à 1 ; et/ou
R₁ représente un groupe choisi parmi notamment un phényle, pyridinyle, pyridazinyle, pyrimidinyle,
ce groupe étant éventuellement substitué par un groupe R₄ ; et/ou
R₄ représente un groupe choisi parmi notamment un phényle, benzofuranyle, naphtyle ; le groupe R₄ pouvant être substitué par un ou plusieurs groupes R₃ identiques ou différents l'un de l'autre, plus particulièrement par un ou deux groupes R₃ identiques ou différents l'un de l'autre ; et/ou
R₂ représente un groupe de formule générale CHR₅CONHR₆; et/ou
R₃ représente un atome d'halogène, plus particulièrement un chlore, un brome ou un fluor, ou un groupe nitro, C₁₋₆-alkyle, plus particulièrement un méthyle, isopropyle, C₁₋₆-alcoxy, plus particulièrement un méthoxy, éthoxy, C₁₋₆-fluoroalkyle, plus particulièrement un CF₃, C₁₋₆-fluoroalcoxy, plus particulièrement un OCF₃, -O-(C₁₋₃-alkylène)-O-, plus particulièrement un -O-CH₂-O-, benzyloxy ; et/ou
R₅ représente un atome d'hydrogène ;et/ou
R₆ représente un atome d'hydrogène ou un groupe C₁₋₆-alkyle, plus particulièrement un méthyle ou un éthyle.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone,
- alkyle, un groupe aliphatique saturé, linéaire ou ramifié, par exemple un groupe C₁₋₃-alkyle représente une chaîne carbonée de 1 à 3 atomes de carbone, linéaire ou ramifiée, plus particulièrement un méthyle, éthyle, propyle, 1-méthyléthyle ,
- alkylène, un groupe alkyle divalent saturé, linéaire
ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ,
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle,
- alcoxy, un groupe -O-alkyle à chaîne aliphatique saturée, linéaire ou ramifiée ,
- thioalkyle, un groupe -S-alkyle à chaîne aliphatique saturée, linéaire ou ramifiée ,
- fluoroalkyle, un groupe alkyle dont un ou plusieurs atomes d'hydrogène ont été substitués par un atome de fluor,
- fluoroalcoxy, un groupe alcoxy dont un ou plusieurs atomes d'hydrogène ont été substitués par un atome de fluor,
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

Ainsi une première méthode de préparation (schéma 1) consiste à faire réagir une amine de formule générale (II), dans laquelle R₁ et m sont tels que définis dans la formule générale (I), avec un carbonate de formule générale (III), dans laquelle Z représente un atome d'hydrogène ou un groupement nitro et R₂ est tel que défini dans la formule générale (I), dans un solvant tel que le toluène ou le dichloroéthane, à une température comprise entre 0 et 80°C.

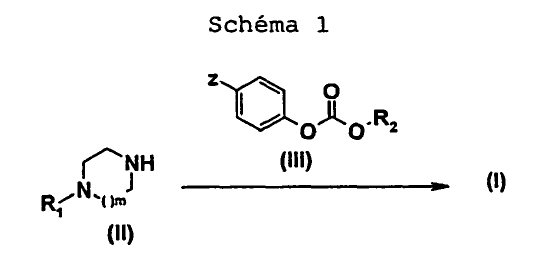

Les carbonates de formule générale (III) peuvent être préparés selon toute méthode décrite dans la littérature, par exemple par réaction d'un alcool de formule générale HOR₂ avec le chloroformiate de phényle ou de 4-nitrophényle, en présence d'une base telle que la triéthylamine ou la diisopropyléthylamine, à une température comprise entre 0°C et la température de reflux du solvant.

Selon une seconde méthode (schéma 2), les composés de formule générale (I) peuvent être préparés en faisant réagir une amine de formule générale (II), telle que définie ci-dessus, avec un carbonate de formule générale (IIIa) dans laquelle Z représente un atome d'hydrogène ou un groupe nitro, R₅ est tel que défini dans la formule générale (I) et R représente un groupe méthyle ou éthyle.

Le carbamate-ester de formule générale (Ia) ainsi obtenu est ensuite transformé en composé de formule générale (I), par aminolyse au moyen d'une amine de formule générale R₆NH₂ dans laquelle R₆ est tel que défini dans la formule générale (I). La réaction d'aminolyse peut être réalisée dans un solvant tel que le méthanol ou un mélange de solvants tels que le méthanol et le tétrahydrofurane ou le méthanol et le dioxane.

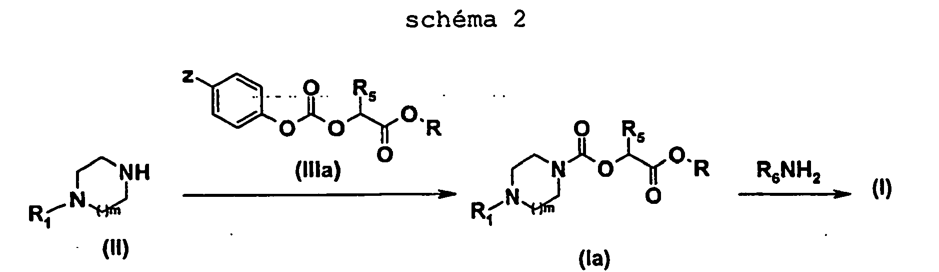

Les carbonates de formule générale (IIIa) peuvent être préparés selon toute méthode décrite dans la littérature, par exemple par réaction d'un alcool de formule générale HOCHR₅COOR où R représente un groupe méthyle ou éthyle, avec le chloroformiate de phényle ou de 4-nitrophényle, en présence d'une base telle que la triéthylamine ou la diisopropyléthylamine.

Les composés de formule générale (I), dans laquelle R₁ représente un groupe substitué par un groupe R₃ de type C₁₋₆-alkyle, C₃₋₇-cycloalkyle ou C₃₋₇-cycloalkyle-C₁₋₃-alkylène, ou par un groupe R₄ tel que défini dans la formule générale (I), peuvent être également préparés par une réaction de type Suzuki réalisée sur les composés de formule générale (I) correspondants, pour lesquels R₁ est substitué par un atome de chlore, de brome, d'iode ou par un groupement triflate dans la position où le groupe R₃ ou R₄ doit être introduit, par exemple au moyen d'un acide boronique d'alkyle, de cycloalkyle, d'aryle ou d'hétéroaryle.

Pour les composés de formule générale (I), dans laquelle R₁ représente un groupe substitué par un groupe R₃ de type C₁-6-alkyle, C₃₋₇-cycloalkyle ou C₃₋₇-cycloalkyle-C₁₋₃-alkylène ou par un groupe R₄ tel que défini dans la formule générale (I), et R₂ représente plus particulièrement un groupement de formule générale CHR₅CONHR₆, la réaction de Suzuki décrite ci-dessus peut-être effectuée sur le carbamate-ester de formule générale (Ia) telle que définie ci-dessus. L'action d'une amine de formule générale R₆NH₂ telle que définie ci-dessus sur le carbamate-ester ainsi obtenu permet d'obtenir les composés de formule générale (I) .

Les composés de formule générale (II), quand leur mode de préparation n'est pas décrit, sont disponibles dans le commerce ou décrits dans la littérature, ou bien peuvent être préparés selon des méthodes qui y sont décrites ou qui sont connues de l'homme du métier.
Les amines de formule générale R₆NH₂ sont disponibles dans le commerce.

L'invention, selon un autre de ses aspects, a également pour objet les composés de formule (Ia). Ces composés sont utiles comme intermédiaires de synthèse des composés de formule (I).

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius. Les numéros indiqués entre parenthèses dans les titres des exemples correspondent à ceux de la 1ère colonne du tableau ci-après.

### Exemple 1 (Composé N°44)

### 4-{4'-[(trifluorométhyl)oxy]-4-biphénylyl}-1-pipérazinecarboxylate de 2-(méthylamino)-2-oxoéthyle

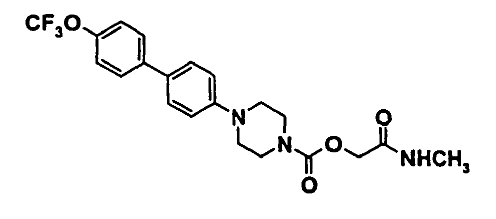

### 1.1.[(phényloxycarbonyl)oxy]acétate d'éthyle

A une solution de 25 g (240 mmoles) de glycolate d'éthyle et de 55 ml (315 mmoles) de diisopropyléthylamine dans 500 ml de toluène, on ajoute lentement à température ambiante 32 ml (256 mmoles) de chloroformiate de phényle. On poursuit l'agitation à température ambiante pendant 2 heures.
On sépare le sel formé et on concentre le filtrat sous pression réduite.
On obtient 53,7 g de produit huileux que l'on utilise tel quel dans l'étape suivante.

### 1.2. 4-(4-bromophényl)-1-pipérazine carboxylate de 2-(éthyloxy)-2-oxoéthyle

On chauffe à 80°C pendant 12 heures, une solution de 5, 81 g (24,08 mmoles) de 1-(4-bromophényl)pipérazine et de 6 g (26, 76 mmoles) de [(phényloxycarbonyl)oxy] acétate d'éthyle, obtenu à l'étape 1.1., dans 50 ml de toluène.
On laisse revenir à température ambiante, on concentre sous pression réduite puis on purifie le résidu ainsi obtenu par chromatographie sur gel de silice en éluant avec un mélange 20/80 puis 30/70 d'acétate d'éthyle et de cyclohexane.
On obtient ainsi 7,75 g de produit pur sous forme d'huile qui cristallise à température ambiante.
PF (°C) : 80-82°C

### 1.3. 4-{4'-(trifluorométhyl)oxy]-4-biphénylyl}-1-pipérazinecarboxylate de 2-(éthyloxy)-2-oxoéthyle

Sous atmosphère inerte, on introduit 2 g (5,39 mmole) de 4-(4-bromophényl)-1-pipérazinecarboxylate de 2-(éthyloxy)-2-oxoéthyle, obtenu à l'étape 1.2., 3, 33 g (16,16 mmoles) d'acide 4-(trifluorométhoxy)phényle boronique et 4,57 g (21,55 mmoles) de phosphate de potassium hydraté en suspension dans 18 ml de 1,2-diméthoxyéthane. On ajoute ensuite 0,62 g (0, 54 mmole) de palladium tétrakis (triphénylphosphine) . On porte ensuite le mélange réactionnel à environ 80°C pendant 12 heures.
On concentre sous pression réduite. On reprend le résidu avec de dichlorométhane et de l'eau, on sépare la phase aqueuse, on l'extrait deux fois avec du dichlorométhane, on sèche les phases organiques réunies sur sulfate de sodium et on concentre le filtrat sous pression réduite. On purifie le résidu ainsi obtenu par chromatographie sur gel de silice en éluant avec un mélange 30/70 d'acétate d'éthyle et de cyclohexane.
On obtient 1, 65 g de produit sous forme de solide blanc.
PF (°C) : 112-116°C

### 1.4. 4-{4'-[(trifluorométhyl)oxy]-4-biphénylyl}-1-pipérazinecarboxylate de 2-(méthylamino)-2-oxoéthyle

A une solution de 1,60 g (3,54 mmoles) de 4-{4'-[(trifluorométhyl)oxy]-4-biphénylyl}-1-pipérazine carboxylate de 2-(éthyloxy)-2-oxoéthyle, préparé à l'étape 1.3., dans 14 ml de méthanol, on ajoute 7,10 ml (14,15 mmoles) d'une solution de méthylamine (2M) dans le tétrahydrofurane. On poursuit l'agitation à température ambiante pendant 12 heures.
Après concentration sous pression réduite, on purifie le résidu obtenu par chromatographie sur gel de silice en éluant avec un mélange 97/3 de dichlorométhane et de méthanol. On obtient un solide qu'on recristallise dans un mélange d'acétate d'éthyle et de diisopropyléther.
On obtient ainsi 0,86 g de produit pur sous forme de solide blanc.
LC-MS : M+H = 438
PF (°C) : 187-189°C
RMN¹H (CDCl₃) δ (ppm) : 2,90 (d, 3H) ; 3,25 (m, 4H) ; 3,70 (m, 4H) ; 4,60 (s, 2H) ; 6,10 (large s, 1H) ; 7,0 (d, 2H) ; 7,30 (d, 2H) ; 7,50 (d, 2H) ; 7,60 (d, 2H).

### Exemple 2 (Composé N°37)

### 4-[3'-(trifluorométhyl)-4-biphénylyl]-1-pipérazine carboxylate de 2-(méthylamino)-oxoéthyle

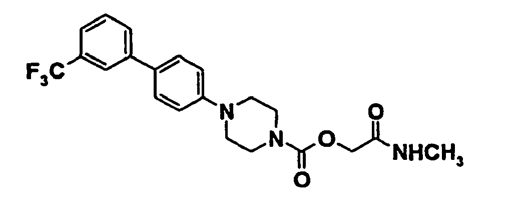

### 2.1. 4-nitrophényle carbonate de 2-(méthylamino)-2-oxoéthyle

A une suspension de 2,62 g (29,4 mmoles) de 2-hydroxy-*N-*méthylacétamide et de 16,5 g (58,7 mmoles) de diisopropyléthylamine supportée (Ps-DIEA d'Argonaut, charge = 3,56 mmoles/g) dans 250 ml de dichlorométhane, on ajoute par petites portions et à température ambiante 5,93 g (29,4 mmoles) de chloroformiate de 4-nitrophényle. On poursuit l'agitation orbitalaire à température ambiante pendant 16 heures.
On filtre la résine,-on rince avec 150 ml de dichlorométhane et on concentre le filtrat sous pression réduite.
On obtient 6 g de produit sous forme de solide jaune-claire (pureté estimée à 70%) que l'on utilise tel quel dans l'étape suivante.

### 2.2. 4-(4-bromophényl)-1-pipérazinecarboxylate de 2-(méthylamino)-2-oxoéthyle

A une solution de 1,47 g (4 mmoles) de 4-nitrophényle carbonate de 2-(méthylamino)-2-oxoéthyle, obtenu à l'étape 2.1., dans 18 ml de 1,2-dichloroéthane, on ajoute 1,17 g (4, 85 mmoles) de 1-(4-bromophényl)pipérazine. On chauffe ce mélange réactionnel à 65°C pendant 2,25 heures.
On laisse revenir à température ambiante puis on concentre sous pression réduite. On reprend le résidu huileux jaune avec du dichlorométhane et on lave successivement avec de la soude (1N), de l'eau, une solution aqueuse d'acide citrique à 5%, de l'eau puis de la saumure. On sèche cette phase organique sur sulfate de sodium et on concentre sous pression réduite. Après lavage par le diisopropyléther, on obtient 1,3 g de produit sous forme de solide blanc.

### 2.3. Synthèse du catalyseur au palladium greffé sur la résine Merrifield

Sous atmosphère inerte, à une suspension de 5 g (3.5 mmoles) de résine Merrifield (Fluka, 200-400 Mesh, recticulée avec 2% de divinylbenzène (DVB), Charge = 0,7 mmole/g) dans 50 ml de tétrahydrofurane (THF) anhydre, on introduit 54,6 ml (27,3 mmoles) d'une solution de diphénylphosphide de lithium commercialisée à 0,5 M dans le THF. On poursuit l'agitation orbitalaire à température ambiante pendant 24 heures puis on ajoute 60 ml d'acétone et 20 ml d'eau. On filtre la résine et on la lave successivement avec de l'eau, de l'acétone, du THF, un mélange THF/H₂O (2/1), du THF, du toluène, du dichlorométhane et de l'éther éthylique puis on la sèche sous vide pendant 2 heures.
On porte à 70°C pendant 24 heures une suspension de la résine ainsi obtenue dans 47 ml d'éthanol et 23 ml de toluène. Après filtration, on lave la résine successivement avec de l'acétone, du THF et de l'éther éthylique. Au total, ce traitement est répété quatre fois pour éliminer les fractions solubles du polymère. On sèche la résine ainsi obtenue sous vide pendant 2 heures.
A une suspension de cette résine dans 60 ml de toluène, on ajoute 0,18 g (0,16 mmole) de palladium tétrakis (triphénylphosphine) et on porte ce mélange réactionnel à 95°C pendant 24 heures.
On laisse revenir à température ambiante, on filtre la résine et on la lave successivement avec de l'acétone, du THF puis de l'éther éthylique. On obtient 5,135 g de résine que l'on utilise telle quelle dans l'étape suivante.

### 2.4. 4-[3'-(trifluorométhyl)-4-biphénylyl]-1-pipérazinecarboxylate de 2-(méthylamino)-2-oxoéthyle

On introduit 0,18 g (0,5 mmole) de 4-(4-bromophényl)-1-pipérazinecarboxylate de 2-(méthylamino)-2-oxoéthyle, obtenu à l'étape 2. 2., 0,21 g (1, 1 mmoles) d'acide 3-(trifluorométhyl)phényle boronique et 0,16 g (1,5 mmoles) de carbonate de sodium en suspension dans 3 ml de toluène et 0,3 ml d'éthanol. On ajoute ensuite 0,14 g (~10% molaire) de catalyseur au palladium supporté, obtenu à l'étape 2.3. et on poursuit l'agitation orbitalaire à 80°C pendant 48 heures.
On laisse revenir à température ambiante, on filtre la résine, on rince avec du dichlorométhane et on concentre le filtrat sous pression réduite.
On reprend le résidu dans 5 ml de dichlorométhane et on lave avec de l'eau puis une solution aqueuse saturée de bicarbonate de sodium. On filtre la phase organique sur une cartouche hydrophobe puis on concentre le filtrat sous pression réduite.
On obtient un résidu huileux qui cristallise dans le diisopropyléther.
On obtient 0,15 g de cristaux blancs.
LC-MS : M+H = 422
PF (°C) : 129-130°C
RMN¹H (CDCl₃) δ (ppm) : 2,95 (d, 3H) ; 3,20-3,35 (m, 4H) ; 3,65-3,80 (m, 4H) ; 4,65 (s, 2H) ; 6,05 (large s, 1H) ; 7,05 (d, 2H) ; 7,50-7,60 (m, 4H) ; 7,65-7,80 (m, 2H)

### Exemple 3 (Composé N°76)

### 4-{5-[3-(trifluorométhyl)phényl]-2-pyridinyl}-1-pipérazinecarboxylate de 2-(méthylamino)-2-oxoéthyle

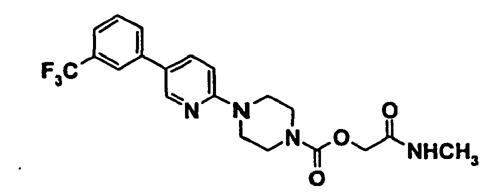

### 3.1. 4-(5-bromo-2-pyridinyl)-1-pipérazine carboxylate de 1,1-diméthyléthyle

Dans un autoclave, on introduit 29,2 g (157 mmoles) de 1-pipérazinecarboxylate de 1,1-diméthyléthyle, 37 g (157 mmoles) de 2, 5-dibromopyridine et 21, 7 g (157 mmoles) de carbonate de potassium en suspension dans 27 ml de diméthylsulfoxyde (DMSO). On chauffe ensuite à 150°C pendant 21 heures.
On laisse revenir à température ambiante, on reprend le mélange réactionnel par de l'acétate d'éthyle et de l'eau puis on sépare l'insoluble par filtration. On sépare la phase aqueuse, on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite. On purifie le résidu obtenu par chromatographie sur gel de silice en éluant avec un mélange 99/1 de dichlorométhane et de méthanol.
On obtient ainsi 44 g de produit sous forme de solide blanc.
PF(°C) : 83-85°C

### 3.2. 1-(5-bromo-2-pyridinyl)pipé razine

A une solution de 18,60 g (54, 40 mmoles) de 4-(5-bromo-2-pyridinyl)-1-pipérazine-carboxylate de 1,1-diméthyléthyle, obtenu à l'étape 3.1., dans 100 ml de 1,4-dioxane, on ajoute à température ambiante, 49 ml (272 mmoles) d'une solution d'acide chlorhydrique (6N) dans l'isopropanol. On porte ensuite le mélange réactionnel à environ 60°C pendant 3 heures.
On concentre à sec sous pression réduite. On reprend le dichlorhydrate obtenu dans 200 ml de dichlorométhane et 200 ml d'eau puis on additionne par petites portions, sous agitation, 10 g d'hydrogénocarbonate de sodium. On décante, on extrait deux fois la phase aqueuse par du dichlorométhane, on lave les phases organiques réunies avec une solution aqueuse saturée en chlorure de sodium, on les sèche sur sulfate de sodium et on concentre sous pression réduite.
On obtient 12 g de produit sous forme de solide blanc.
PF (°C) : 72°C

### 3.3. 4-(5-bromo-2-pyridinyl)-1-pipérazinecarboxylate de 2-(éthyloxy)-2-oxoéthyle

On procède comme décrit dans l'exemple 1 (étape 1.2.). A partir de 6 g (24,80 mmoles) de 1-(5-bromo-2-pyridinyl)pipérazine, obtenu à l'étape 3.2., et de 10, 88 g (48, 52 mmoles) de [(phényloxycarbonyl)oxy]acétate d'éthyle, préparé à l'étape 1.1. de l'exemple 1, et après chromatographie sur gel de silice en éluant avec un mélange 15/85 puis 30/70 d'acétate d'éthyle et de cyclohexane, on obtient 6,70 g de produit sous forme d'huile qui cristallise en solide blanc.

### 3.4. 4-{5-[3-(trifluorométhyl)phényl]-2-pyridinyl}-1-pipérazinecarboxylate de 2-(éthyloxy)-2-oxoéthyle

On procède suivant le mode opératoire décrit dans l'exemple 1 (étape 1.3.). A partir de 3 g (8,06 mmoles) de 4-(5-bromo-2-pyridinyl)-1-pipérazine-carboxylate de 2-(éthyloxy)-2-oxoéthyle, obtenu à l'étape 3.3., de 4,59 g (24,17 mmoles) d'acide 3-(trifluorométhyl)phényle boronique, de 6,84 g (32,23 mmoles) de phosphate de potassium hydraté et de 0,93 g (0,806 mmole) de palladium tétrakis(triphénylphosphine), et après chromatographie sur gel de silice en éluant avec un mélange 30/70 d'acétate d'éthyle et de cyclohexane, on obtient 2,22 g de produit sous forme de solide blanc.

### 3.5. 4-{5-[3-(trifluorométhyl)phényl]-2-pyridinyl}-1-pipérazinecarboxylate de 2-(méthylamino)-2-oxoéthyle

On procède suivant le mode opératoire décrit dans l'exemple 1 (étape 1.4.) . A partir de 1,50 g (3,43 mmoles) de 4-{5-[3-(trifluorométhyl)phényl]-2-pyridinyl}-1-pipérazinecarboxylate de 2-(éthyloxy)-2-oxoéthyle, obtenu à l'étape 3.4., et de 8,6 ml (17,15 mmoles) d'une solution de méthylamine (2M) dans le tétrahydrofurane, et après chromatographie sur gel de silice en éluant avec un mélange 97/3 de dichlorométhane et de méthanol, suivie d'un lavage par le diisopropyléther, on obtient 1,18 g de produit sous forme de solide blanc.
LC-MS : M+H = 423
PF(°C) : 158-160°C
RMN¹H (CDCl₃) δ (ppm) : 2,90 (d, 3H) ; 3,75 (large s, 8H) ; 4,65 (s, 2H) ; 6,05 (large s, 1H) ; 6,75 (d, 1H) ; 7,50-7,80 (massif, 5H) ; 8, 50 (d, 1H).

### Exemple 4 (Composé N°79)

### 4-{5-[4-(trifluorométhyl)phényl]-2-pyridinyl}-1-pipérazinecarboxylate de 2-(méthylamino)-2-oxoéthyle

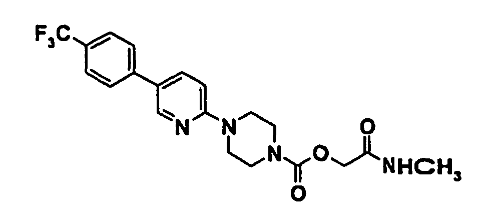

### 4.1. 4-{5-[4-(trifluorométhyl)phényl]-2-pyridinyl}-1-pipérazinecarboxylate de 2-(éthyloxy)-2-oxoéthyle

On procède suivant le mode opératoire décrit dans l'exemple 1 (étape 1.3.). A partir de 4 g (10,75 mmoles) de 4-(5-bromo-2-pyridinyl)-1-pipérazine carboxylate de 2-(éthyloxy)-2-oxoéthyle, obtenu à l'étape 3.3. de l'exemple 3, de 5,50 g (28,96 mmoles) d' acide 4-(trifluorométhyl)phényle boronique, de 9,12 g (42,99 mmoles) de phosphate de potassium hydraté et de 1,24 g (1,07 mmoles) de palladium tétrakis(triphénylphosphine), et après chromatographie sur gel de silice en éluant avec un mélange 30/70 d'acétate d'éthyle et de cyclohexane, on obtient 2,78 g de produit sous forme de solide blanc.

### 4.2. 4-{5-[4-(trifluorométhyl)phényl]-2-pyridinyl}-1-pipérazinecarboxylate de 2-(méthylamino) -2-oxoéthyle

On procède suivant le mode opératoire décrit dans l'exemple 1 (étape 1.4.). A partir de 2,77 g (6,33 mmoles) de 4-{5-[4-(trifluorométhyl)phényl]-2-pyridinyl}-1-pipérazinecarboxylate de 2-(éthyloxy)-2-oxoéthyle, obtenu à l'étape 4.1., et de 15,80 ml (31,67 mmoles) d'une solution de méthylamine (2M) dans le tétrahydrofurane, et après chromatographie sur gel de silice en éluant avec un mélange 97/3 de dichlorométhane et de méthanol, suivie d'une recristallisation dans l'acétate d'éthyle, on obtient 1,69 g de produit sous forme de solide blanc.
LC-MS : M+H = 423
PF(°C) : 206-209°C
RMN¹H (CDCl₃) δ (ppm) : 2, 90 (d, 3H) ; 3,70 (large s, 8H) ; 4,65 (s, 2H) ; 6,05 (large s, 1H) ; 6,75 (d, 1H) ; 7, 60-7, 75 (m, 4H) ; 7, 80 (dd, 1H) ; 8,50 (d, 1H).

### Exemple 5 (Composé N°83)

### 4-(5-{4-[(trifluorométhyl)oxy]phényl}-2-pyridinyl)-1-pipérazinecarboxylate de 2-(méthylamino)-2-oxoéthyle

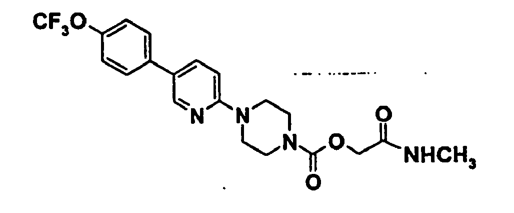

### 5.1. 2-(méthylamino)-2-oxoéthyl 1,4-pipérazine dicarboxylate de 1,1-diméthyléthyle

A une solution refroidie à 0°C de 1,1 g (3 mmoles) de 4-nitrophényle carbonate de 2-(méthylamino)-2-oxoéthyle, préparé à l'étape 2.2. de l'exemple 2, dans 10 ml de 1,2-dichloroéthane, on ajoute goutte à goutte et à environ 0°C, une solution de 0,53 g (2,85 mmoles) de 1-pipérazinecarboxylate de 1,1-diméthyléthyle dans 5 ml de 1,2-dichloroéthane. On poursuit l'agitation à 0°C pendant 1 heure, puis à température ambiante pendant 3 heures.
On concentre sous pression réduite et on purifie le résidu ainsi obtenu par chromatographie sur gel de silice en éluant avec un mélange 20/80 d'acétate d'éthyle et de cyclohexane. On augmente progressivement le gradient pour terminer l'élution avec l'acétate d'éthyle.
On obtient un résidu huileux qui cristallise dans le diisopropyléther.
On obtient 0,61 g de produit sous forme de solide blanc que l'on utilise tel quel dans l'étape suivante.

### 5.2. Chlorhydrate de 1-pipérazinecarboxylate de 2-(méthylamino)-2-oxoéthyle

A une solution de 2,68 g (8,9 mmoles) de 1,1-diméthyléthyl 1,4-pipérazine dicarboxylate de 2-(méthylamino)-2-oxoéthyle, obtenu selon l'étape 5. 1., dans 25 ml de dichlorométhane, on ajoute 25 ml d'une solution d'acide chlorhydrique 6 N dans l'isopropanol. On poursuit l'agitation à température ambiante pendant 1 heure.
On élimine la phase organique par filtration à travers une cartouche hydrophobe et on concentre la phase aqueuse acide sous pression réduite.
Après cristallisation dans l'isopropanol, on obtient 2,05 g de produit sous forme de solide blanc que l'on utilise tel quel dans l'étape suivante.
PF (°C) : 167-169°C

### 5.3. 4-(5-nitro-2-pyridinyl)-1-pipérazinecarboxylate de 2-(méthylamino)-2-oxoéthyle

A une solution de 2,05 g (8,62 mmoles) de chlorhydrate de 1-pipérazinecarboxylate de 2-(méthylamino)-2-oxoéthyle, obtenu à l'étape 5.2., et 3,85 ml (22,4 mmoles) de N,N diisopropyléthylamine dans 55 ml de 1,2-dichloroéthane, on ajoute 1,84 g (11,6 mmoles) de 2-chloro-5-nitropyridine. On porte ce mélange réactionnel à 70°C pendant 5 heures.
On laisse revenir à température ambiante, on concentre sous pression réduite et on purifie le résidu ainsi obtenu par chromatographie sur gel de silice en éluant avec un mélange de 98/2 de dichlorométhane et de méthanol.
On obtient 2,48 g de produit sous forme de solide jaune-claire que l'on utilise tel quel dans l'étape suivante.

### 5.4. 4-(5-amino-2-pyridinyl)-1-pipérazinecarboxylate de 2-(méthylamino)-2-oxoéthyle

A une suspension de 0,64 g (1, 98 mmole) de 4-(5-nitro-2-pyridinyl)-1-pipérazinecarboxylate de 2-(méthylamino)-2-oxoéthyle, préparé à l'étape 5.3., dans 90 ml d'acétate d'éthyle, on ajoute 0,24 g de palladium sur charbon à 10%. On poursuit l'agitation à température ambiante sous une atmosphère d'hydrogène de 60 psi pendant 14 heures. Après filtration du catalyseur, on concentre le filtrat sous pression réduite et on purifie le résidu ainsi obtenu par chromatographie sur gel de silice en éluant avec un mélange 98/2 de dichlorométhane et de méthanol. On obtient 0,47 g de produit sous forme d'huile violacée que l'on utilise tel quel dans l'étape suivante.

### 5.5. 4-(5-iodo-2-pyridinyl)-1-pipérazinecarboxylate de 2-(méthylamino)-2-oxoéthyle

A une solution refroidie à 0°C de 0,47 g (1,5 mmoles) de 4-(5-amino-2-pyridinyl)-1-pipérazinecarboxylate de 2-(méthylamino)-2-oxoéthyle, préparé à l'étape 5.4., dans 15 ml d'une solution aqueuse d'acide sulfurique (0,33N), on ajoute lentement une solution de 0,16 g (2,2 mmoles) de nitrite de sodium dissout dans 3,5 ml d'eau. On poursuit l'agitation à environ 0°C pendant 0,5 heure et on ajoute lentement 0,83 g (5 mmoles) d'iodure de potassium. On poursuit l'agitation à cette température pendant 0,5 heure puis on porte le mélange réactionnel à 85°C pendant 2 heures.
Après refroidissement à température ambiante, on basifie le milieu réactionnel jusqu'à pH = 14, par addition d'une solution aqueuse saturée de bicarbonate de sodium. On extrait la phase aqueuse trois fois avec du dichlorométhane, on lave les phases organiques réunies avec une solution aqueuse de thiosulfite à 35%, de l'eau, de la saumure et on les sèche sur sulfate de sodium. On concentre le filtrat sous pression réduite et on purifie le résidu ainsi obtenu par chromatographie sur gel de silice en éluant avec un mélange 98/2 de dichlorométhane et de méthanol.
Après lavage par le diisopropyléther, on obtient 0,35 g de produit sous forme de solide beige que l'on utilise tel quel dans l'étape suivante.

### 5.6. 4-(5-(4-[(trifluorométhyl)oxy]phényl)-2-pyridinyl)-1-pipérazinecarboxylate de 2-(méthylamino)-2-oxoéthyle

On procède suivant le mode opératoire décrit dans l'exemple 2 (étape 2.4.). A partir de 0,250 g (0,61 mmole) de 4-(5-iodo-2-pyridinyl)-1-pipérazinecarboxylate de 2-(méthylamino)-2-oxoéthyle, obtenu à l'étape 5.5., de 0,51 g (2, 44 moles) d'acide 4-(trifluorométhoxy) phényle boronique, de 0,61 g (~ 8% molaire) de catalyseur au palladium sur support solide, préparé à l'étape 2.1. de l'exemple 2, et de 2,9 ml (7,32 mmoles) d'une solution aqueuse de carbonate de sodium (2,5M) en suspension dans 12 ml de toluène et 3 ml d'éthanol, et après chromatographie sur gel de silice en éluant avec un mélange 98/2 de dichlorométhane et de méthanol, suivie d'un lavage par le diisopropyléther, on obtient 0,092 g de produit sous forme de solide blanc.
LC-MS : M+H = 439
PF (°C) : 188-190°C
RMN¹H (CDCl₃) (ppm) : 2,90 (d, 3H) ; 4,70 (large s, 8H) ; 4,65 (s, 2H) ; 6,05 (large s, 1H) ; 6,75 (dd, 1H) ; 7,30 (d, 2H) ; 7, 55 (d, 2H) : 7, 75 (dd, 1H) ; 8,45 (dd, 1H).

### Exemple 6 (Composé N°63)

### 4-[5-(2-méthylpropyl)-2-pyridinyl]-1-pipérazine carboxylate de 2-(méthylamino)-2-oxoéthyle

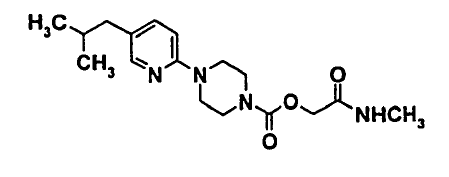

### 6.1. 4-(5-bromo-2-pyridinyl)-1-pipérazinecarboxylate de 2-(méthylamino)-2-oxoéthyle

On procède comme décrit dans l'exemple 1 (étape 1.4.) . A partir de 2,20 g (5,91 mmoles) de 4-(5-bromo-2-pyridinyl)-1-pipérazinecarboxylate de 2-(éthyloxy)-2-oxoéthyle, obtenu à l'étape 3.3. de l'exemple 3, et de 14,80 ml (29,55 mmoles) d'une solution de méthylamine (2M) dans le tétrahydrofurane, et après cristallisation dans le diisopropyléther, on obtient 1,974 g de produit pur sous forme de solide blanc.

### 6.2. 4-[5-(2-méthylpropyl)-2-pyridinyl]-1-pipérazine carboxylate de 2-(méthylamino)-2-oxoéthyle

Sous atmosphère inerte, on met 0,88 g (2,47 mmoles) de 4-(5-bromo-2-pyridinyl)-1-pipérazine carboxylate de 2-(méthylamino)-2-oxoéthyle, obtenu à l'étape 6.1., 0,33 g (3,22 mmoles) d'acide isobutylboronique, 1,16 g (5,44 mmoles) de phosphate de potassium hydraté et 0,07 g (0,25 mmole) de tricyclohexylphosphine en suspension dans 11 ml de toluène. On ajoute ensuite 0,028 g (0,12 mmole) de palladium diacétate. On porte ensuite le mélange réactionnel au reflux pendant 3 heures.
On laisse revenir à température ambiante puis on ajoute 15 ml d'eau et 15 ml d'acétate d'éthyle. On sépare les sels par filtration sur fritté, on décante, on extrait la phase aqueuse deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 97/3 de dichlorométhane et de méthanol.
On obtient, après cristallisation dans le diisopropyl éther, 0,17 g de produit sous forme de solide blanc.
LC-MS : M+H = 335
PF(°C) : 127-129°C
RMN¹H (CDCl₃) δ (ppm) : 0,90 (d, 6H) ; 1,80 (m, 1H) ; 2, 35 (d, 2H) ; 2, 90 (d, 3H) ; 3,60 (m, 8H) ; 4,65 (s, 2H) ; 6,10 (large s, 1H) ; 6,60 (d, 1H) ; 7, 35 (dd, 1H) ; 8, 0 (d, 1H).

### Exemple 7 (Composé N°85)

### 4-{6-[3-(trifluorométhyl)phényl]-3-pyridinyl}-1-pipérazinecarboxylate de 2-(méthylamino)-2-oxoéthyle

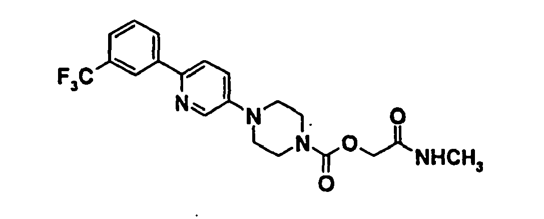

### 7.1. 4-(3-pyridinyl)-1-pipérazinecarboxylate de 1,1-diméthyléthyle

Sous atmosphère inerte, on introduit 7,07 g (44, 74 mmoles) de 3-bromopyridine, 10 g (53,69 mmoles) de 1-pipérazinecarboxylate de 1,1-diméthyléthyle, 6,02 g (62,64 mmoles) de *tert*-butylate de sodium et 0,836 g (1,34 mmole) de (2,2'-*bis*(diphénylphosphino)-1,1'-binaphthyle) (BINAP) en suspension dans 100 ml de toluène. On ajoute ensuite 0,41 g (0,45 mmole) de [dipalladium *tris*(dibenzylidèneacétone)] (Pd₂(dba)₃). On porte ensuite le mélange réactionnel au reflux pendant 22 heures.
On laisse revenir à température ambiante, on sépare les sels par filtration sur fibre de verre puis on concentre le filtrat sous pression réduite. On reprend le résidu avec 100 ml d'acétate d'éthyle et 100 ml d'eau, on sépare la phase aqueuse, on l'extrait plusieurs fois avec de l'acétate d'éthyle, on lave les phases organiques réunies avec une solution aqueuse saturée en chlorure de sodium, on les sèche sur sulfate de sodium et on concentre le filtrat sous pression réduite. On purifie le résidu ainsi obtenu par chromatographie sur gel de silice en éluant avec un mélange 98/2 puis 95/5 de dichlorométhane et de méthanol.
On obtient 9,80 g de produit sous forme d'huile qui cristallise à température ambiante.

### 7.2. 4-(6-bromo-3-pyridinyl)-1-pipérazinecarboxylate de 1,1-diméthyléthyle

A une solution de 4 g (15,19 mmoles) de 4-(3-pyridinyl)-1-pipérazinecarboxylate de 1,1-diméthyléthyle, obtenu à l'étape 7.1., dans 50 ml d'acétonitrile, refroidie à environ 0°C, on ajoute par petites portions 2,70 g (15,19 mmoles) de N-bromosuccinimide (NBS). On poursuit l'agitation à 0°C pendant 15 minutes puis à température ambiante pendant 2 heures.
On ajoute au milieu réactionnel 100 ml d'une solution aqueuse d'hydroxyde de sodium (1M) et 100 ml d'acétate d'éthyle. On sépare la phase aqueuse, on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite.
On obtient ainsi 5,16 g de produit sous forme de solide jaune orangé utilisé tel quel dans l'étape suivante.

### 7.3. 1-(6-bromo-3-pyridinyl)pipérazine

A une suspension de 5,16 g (15,08 mmoles) de 4-(6-bromo-3-pyridinyl)-1-pipérazinecarboxylate de 1,1-diméthyléthyle, obtenu à l'étape 7.2., dans 70 ml de dichlorométhane, on ajoute lentement 11,20 ml (150,77 mmoles) d'acide trifluoroacétique. On poursuit l'agitation à température ambiante pendant 16 heures.
On concentre sous pression réduite, on reprend le résidu avec 40 ml de chloroforme puis on additionne lentement 4 ml d'une solution aqueuse d'hydroxyde de sodium (10M). On sépare la phase aqueuse puis on l'extrait deux fois avec de chloroforme. On réunit les phases organiques et on les lave avec une solution aqueuse saturée de chlorure de sodium. On sèche la phase organique sur sulfate de sodium et on concentre le filtrat sous pression réduite.
On obtient ainsi 5,16 g de produit sous forme d'huile orange qui cristallise à température ambiante. Ce produit est utilisé tel quel dans l'étape suivante.

### 7.4. 4-(6-bromo-3-pyridinyl)-1-pipérazinecarboxylate de 2-(éthyloxy)-2-oxoéthyle

On procède comme décrit dans l'exemple 1 (étape 1.2.). A partir de 3,57 g (14,76 mmoles) de 1-(6-bromo-3-pyridinyl)pipérazine, obtenu à l'étape 7.3., et de 3,97 g (17,71 mmoles) de [(phényloxycarbonyl)oxy]acétate d'éthyle, préparé à l'étape 1.1. de l'exemple 1, et après chromatographie sur gel de silice en éluant avec un mélange 99/1 puis 98/2 de dichlorométhane et de méthanol, on obtient 3,75 g de produit sous forme d'huile jaune qui cristallise à température ambiante.

### 7.5. 4-{6-[3-(trifluorométhyl)phényl]-3-pyridinyl}-1-pipérazinecarboxylate de 2-(éthyloxy)-2-oxoéthyle

On procède suivant le mode opératoire décrit dans l'exemple 1 (étape 1.3.). A partir de 1,28 g (3,43 mmoles) de 4-(6-bromo-3-pyridinyl)-1-pipérazine carboxylate de 2-(éthyloxy)-2-oxoéthyle, obtenu à l'étape 7.4., de 1,96 g (10,29 mmoles) d'acide 3-(trifluorométhyl)phényle boronique, de 2,91 g (13,72 mmoles) de phosphate de potassium hydraté et de 0,40 g (0,34 mmole) de palladium tétrakis(triphénylphosphine), et après chromatographie sur gel de silice en éluant avec un mélangé 35/65 d'acétate d'éthyle et de cyclohexane, on obtient 0,98 g de produit pur sous forme d'huile qui cristallise à température ambiante.

### 7.6. 4-{6-[3-(trifluorométhyl)phényl]-3-pyridinyl}-1-pipérazinecarboxylate de 2-(méthylamino)-2-oxoéthyle

On utilise le mode opératoire décrit dans l'exemple 1 (étape 1.4.). A partir de 0,60 g (1,37 mmoles) de 4-{6-[3-(trifluorométhyl)phényl]-3-pyridinyl}-1-pipérazine carboxylate de 2-(éthyloxy)-2-oxoéthyle, obtenu à l'étape 7.5. et de 3,40 ml (6,86 mmoles) d'une solution de méthylamine (2M) dans le tétrahydrofurane, et après chromatographie sur gel de silice en éluant avec un mélange 98/2 puis 97/3 de dichlorométhane et de méthanol, suivie d'un lavage par le diisopropyléther, on obtient 0,36 g de produit pur sous forme de solide blanc.
LC-MS : M+H = 423
PF(°C) : 146-150°C
RMN¹H (CDCl₃) δ (ppm) : 2,90 (d, 3H) ; 3,35 (m, 4H) ; 3,80 (m, 4H) ; 4,65 (s, 2H) ; 6,05 (large s, 1H) ; 7,30 (m, 1H) ; 7,65 (m, 2H) ; 7,70 (d, 1H) ; 8, 10 (d, 1H) ; 8,25 (s, 1H) ; 8,45 (d, 1H) .

### Exemple 8 (Composé N°86)

### 4-{6-[3-(trifluorométhyl)phényl]-3-pyridinyl}-1-pipérazinecarboxylate de 2-amino-2-oxoéthyle

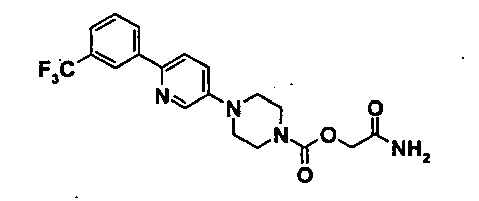

A une solution de 0, 30 g (0,69 mmole) de 4-{6-[3-(trifluorométhyl)phényl]-3-pyridinyl}-1-pipérazine carboxylate de 2-(éthyloxy)-2-oxoéthyle, obtenu à l'étape 7.5. de l'exemple 7, dans 6 ml d'un mélange 1/1 de méthanol et de tétrahydrofurane, on ajoute 5,90 ml (41,40 mmoles) d'une solution d'ammoniac (7N) dans le méthanol. On poursuit l'agitation à température ambiante pendant 22 heures.
Après concentration sous pression réduite, on purifie le résidu obtenu par chromatographie sur gel de silice en éluant avec un mélange 96/4 de dichlorométhane et de méthanol, suivie d'un lavage par le diisopropyléther.
On obtient 0,19 g de produit pur sous forme de solide jaune.
LC-MS : M+H = 409
PF (°C) : 155-157°C
RMN¹H (CDCl₃) δ (ppm) : 3, 35 (m, 4H) ; 3, 75 (m, 4H) ; 4,70 (s, 2H) ; 5, 50 (large s, 1H) ; 6,0 (large s, 1H) ; 7, 30 (m, 1H) ; 7, 55 (m, 2H) ; 7, 70 (d, 1H) ; 8,10 (d, 1H) ; 8,25 (s, 1H) ; 8, 40 (d, 1H).

### Exemple 9 (Composé N°66)

### 4-[6-(2-méthylpropyl)-3-pyridinyl]-1-pipérazinecarboxylate de 2-(méthylamino)-2-oxoéthyle

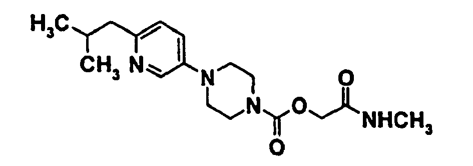

### 9.1. 4-(6-bromo-3-pyridinyl)-1-pipérazinecarboxylate de 2-(méthylamino)-2-oxoéthyle

On utilise le mode opératoire décrit dans l'exemple 1 (étape 1.4.). A partir de 2,35 g (6,32 mmoles) de 2-(éthyloxy)-2-oxoéthyl 4-(6-bromo-3-pyridinyl)-1-pipérazine carboxylate, obtenu à l'étape 7.4. de l'exemple 7, et de 15, 80 ml (31,61 mmoles) d'une solution de méthylamine (2M) dans le tétrahydrofurane, et après chromatographie sur gel de silice en éluant avec un mélange 98/2 puis 97/3 de dichlorométhane et de méthanol, on obtient 1,779 g de produit sous forme de solide blanc.
PF(°C): 164°C

### 9.2. 4-[6-(2-méthylpropyl)-3-pyridinyl]-1-pipérazine carboxylate de 2-(méthylamino)-2-oxoéthyle

Sous atmosphère inerte, on introduit 1,25 g (3,50 mmoles) de 4-(6-bromo-3-pyridinyl)-1-pipérazine carboxylate de 2-(méthylamino)-2-oxoéthyle, préparé à l'étape 9.1., et 0,12 g (0,17 mmole) de palladium dichloro bis (triphénylphosphine) (Pd(PPh₃)₂Cl₂) en suspension dans 7 ml de tétrahydrofurane. On ajoute ensuite 17,50 ml (8,74 mmoles) d'une solution de bromoisobutyl)zinc (0,5M) dans le tétrahydrofurane. On poursuit l'agitation à température ambiante pendant 19 heures.

On verse le mélange réactionnel dans 25 ml d'eau et 25 ml d'acétate d'éthyle. On filtre l'insoluble sur fibre de verre. On décante, on extrait deux fois la phase aqueuse par de l'acétate d'éthyle, on sèche les phases organiques réunies sur sulfate de sodium et on concentre le filtrat sous pression réduite. On purifie le résidu ainsi obtenu par chromatographie sur gel de silice en éluant avec un mélange 95/5 de dichlorométhane et de méthanol, suivie d'une cristallisation dans le diisopropyléther.
On obtient 0,36 g de produit pur sous forme de solide marron.
LC-MS : M+H = 335
PF(°C) . 87-89°C
RMN¹H (CDCl₃) δ (ppm) : 0,90 (d, 6H) ; 2,05 (m, 1H) ; 2,60 (d, 2H) ; 2,90 (d, 3H) ; 3,20 (m, 4H) ; 3,70 (m, 4H) ; 4,65 (s, 2H) ; 6,05 (large s, 1H) ; 7,0 7,20 (m, 2H) ; 8,25 (d, 1H).

### Exemple 10 (Composé N°87)

### 4-{6-[3-(trifluorométhyl)phényl]-3-pyridazinyl}-1-pipérazinecarboxylate de 2-(méthylamino)-2-oxoéthyle

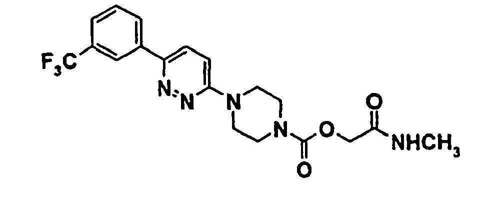

### 10.1. 4-(6-chloro-3-pyridazinyl)-1-pipérazinecarboxylate de 2-(éthyloxy)-2-oxoéthyle

On procède comme décrit dans l'exemple 1 (étape 1.2.). A partir de 1,60 g (8,05 mmoles) de 3-chloro-6-(1-pipérazinyl)pyridazine (J. Med. Chem., 18, 2002, 4011-4017) et de 1,99 g (8,86 mmoles) de [(phényloxy carbonyl)oxy]acétate d'éthyle, préparé à l'étape 1.1. de l'exemple 1, et après chromatographie sur gel de silice en éluant avec un mélange 98/2 de dichlorométhane et de méthanol, on obtient 1,70 g de produit sous forme de solide blanc.
PF (°C) : 149-151°C

### 10.2. 4-{6-[3-(trifluorométhyl)phényl]-3-pyridazinyl}-1-pipérazinecarboxylate de 2-(éthyloxy)-2-oxoéthyle

On procède suivant le mode opératoire décrit dans l'exemple 1 (étape 1.3.). A partir de 1,15 g (3,50 mmoles) de 4-(6-chloro-3-pyridazinyl)-1-pipérazine carboxylate de 2-(éthyloxy)-2-oxoéthyle, obtenu à l'étape 10.1., de 1,99 g (10,49 mmoles) d'acide 3-(trifluorométhyl)phényle boronique, de 2,97 g (13,99 mmoles) de phosphate de potassium hydraté et de 0,40 g (0, 35 mmole) de palladium tétrakis(triphényl phosphine), et après chromatographie sur gel de silice en éluant avec un mélange 35/65 puis 45/55 d'acétate d'éthyle et de cyclohexane, on obtient 0,67 g de produit pur sous forme de solide.
PF (°C) : 126-128°C

### 10.3. 4-{6-[3-(trifluorométhyl)phényl]-3-pyridazinyl}-1-pipérazinecarboxylate de 2-(méthylamino)-2-oxoéthyle

On utilise le mode opératoire décrit dans l'exemple 1 (étape 1.4.). A partir de 0,66 g (1,51 mmoles) de 4-{6-[3-(trifluorométhyl)phényl]-3-pyridazinyl}-1-pipérazine carboxylate de 2-(éthyloxy)-2-oxoéthyle, obtenu à l'étape 10.2. et de 3 ml (6,02 mmoles) d'une solution de méthylamine (2M) dans le tétrahydrofurane, et après chromatographie sur gel de silice en éluant avec un mélange 96/4 de dichlorométhane et de méthanol, suivie d'un lavage par le diisopropyléther, on obtient 0,50 g de produit sous forme de solide blanc.
LC-MS : M+H = 424
PF(°C) : 151-153°C
RMN¹H (DMSO) δ (ppm) : 2,60 (d, 3H) ; 3,55 (m, 4H) ; 3,75 (m, 4H) ; 4,45 (s, 2H) ; 7,40 (d, 1H) ; 7,80 (m, 3H) ; 8,10 (d, 1H) ; 8,35 (m, 2H).

### Exemple 11 (Composé N°103)

### 4-(5-{4-[(trifluorométhyl)oxy]phényl}-2-pyridinyl)-1,4-diazépane-1-carboxylate de 2-(méthylamino)-2-oxoethyle

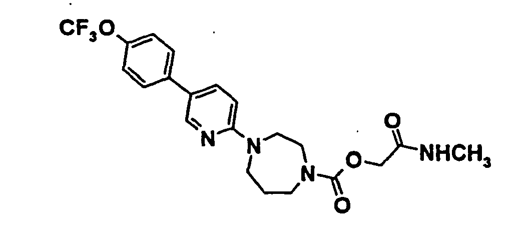

### 11.1. 4-(5-bromo-2-pyridinyl)-1,4-diazépane-1-carboxylate de 1,1-diméthyléthyle

Dans un autoclave, on introduit 1,03 g (5 mmoles) de 1,4-diazépane-1-carboxylate de 1,1-diméthyléthyle, 1,19 g (5 mmoles) de 2,5-dibromopyridine et 0,7 g (5 mmoles) de carbonate de potassium en suspension dans 0,90 ml de diméthylsulfoxyde (DMSO). On chauffe à 150°C pendant 22 heures.
On laisse revenir à température ambiante, on reprend le le mélange réactionnel par de l'acétate d'éthyle, on lavé avec de l'eau puis de la saumure, et on sèche sur sulfate de sodium. On concentre le filtrat sous pression réduite et on purifie le résidu ainsi obtenu par chromatographie sur gel de silice en éluant avec un mélange 99,5/0,5 de dichlorométhane et de méthanol.
On obtient 1,63 g de produit sous forme d'huile que l'on utilise tel quel dans l'étape suivante.

### 11.2. 1-(5-bromo-2-pyridinyl)-1,4-diazépane

A une solution de 1,63 g (4, 4 mmoles) de 4-(5-bromo-2-pyridinyl)-1,4-diazépane-1-carboxylate de 1,1-diméthyléthyle, obtenu à l'étape 11.1., dans 12 ml de dioxane et 4 ml d'éthanol, on ajoute 6 ml d'une solution d'acide chlorhydrique (6N) dans l'isopropanol. On porte ce mélange réactionnel à 70°C pendant 3 heures.
On laisse revenir à température ambiante puis on concentre sous pression réduite. On obtient 1,32 g de solide blanc après cristallisation dans l'acétone.
On reprend ces cristaux dans 10 ml de dichlorométhane et on basifie le milieu réactionnel jusqu'à pH = 14, par addition d'une solution d'ammoniac à 28%. On récupère la phase organique par filtration à travers une cartouche hydrophobe et on concentre le filtrat sous pression réduite.
On obtient 0,96 g de produit sous forme d'huile que l'on utilise tel quel dans l'étape suivante.

### 11.3. 4-(5-bromo-2-pyridinyl)-1,4-diazépane-1-carboxylate de 2-(méthylamino)-2-oxoéthyle

On procède comme décrit dans l'exemple 1 (étape 1.2.). A partir de 0,95 g (3,70 mmoles) de 1-(5-bromo-2-pyridinyl)-1,4-diazépane, obtenu à l'étape 11.2., et de 0,94 g (3,70 mmoles) de 2-(méthylamino)-2-oxoéthyl 4-nitrophényle carbonate, préparé à l'étape 2.2. de l'exemple 2, et après chromatographie sur gel de silice en éluant avec un mélange 30/70 d'acétate d'éthyle et de cyclohexane puis avec un mélange 95/5 de dichlorométhane et de méthanol, suivie d'une cristallisation dans le diisopropyléther, on obtient 0,97 g de produit sous forme de solide blanc.

### 11.4. 4-(5-{4-[(trifluorométhyl)oxy]phényl}-2-pyridinyl)-1,4-diazépane-1-carboxylate de 2-(méthylamino)-2-oxoéthyle

Dans un réacteur en Pyrex, on introduit 0,12 g (0,3 mmole) de 4-(5-bromo-2-pyridinyl)-1,4-diazépane-1-carboxylate de 2-(méthylamino)-2-oxoéthyle, préparé à l'étape 11.3., 0,25 g (1,2 mmoles) d'acide 4-(trifluorométhoxy)phényle boronique et 0,9 ml (1,8 mmoles) d'une solution aqueuse de carbonate de sodium (2M), en suspension dans 3,5 ml de toluène et 0,8 ml d'éthanol. On ajoute ensuite 0,07 g (0,06 mmole) de palladium tétrakis (triphénylphospnine) et, après avoir scellé le réacteur, on porte à 150°C pendant 15 minutes sous irradiation micro-onde.
On récupère la phase organique par décantation et on concentre le filtrat sous pression réduite. On purifie le résidu ainsi obtenu par chromatographie sur gel de silice en éluant avec un mélange 30/70/5 d'acétate d'éthyle, de cyclohexane et de méthanol.
Après cristallisation dans le diisopropyléther, on obtient 0,078 g de produit sous forme de solide blanc.
LC-MS : M+H= 452
PF (°C) : 191-193°C
RMN¹H (DMSO) (ppm) : 1,70-2,00 (m, 2H) ; 2,55 (d, 3H) ; 3,25-3,40 (m, 2H) ; 3,40-3,90 (m, 6H) ; 4,35 (d, 2H) ; 6,75 (d, 1H) ; 7,35 (d, 2H) ; 7,70 (large d, 2H+NH) ; 7,80 (dd, 1H) ; 8,45 (d, 1H).

Le tableau 1 qui suit illustre les structures chimiques et les propriétés physiques de quelques composés selon l'invention.
Dans la colonne « base ou sel », « base » représente un composé sous forme de bas libre, alors que « HCl » représente un composé sous forme de chlorhydrate. Dans le tableau, OMe représente un groupe méthoxy.
Dans la colonne « PF(°C) ou M+H », PF(°C) est le point de fusion du composé en degrés Celsius et M+H est la valeur de la masse du composé protonné par un atome d'hydrogène (masse du composé + 1), déterminée par LC-MS (Liquid Chromatography - Mass Spectroscopy).

**Tableau 1**

| | | | | | |
|---|---|---|---|---|---|
| 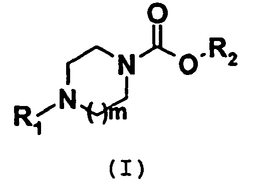 | | | | | |

| **N°** | **R₁** | **m** | **R₂** | **Base ou sel** | **PF (°C) ou M+H** |
|---|---|---|---|---|---|
| 1. | 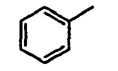 | 1 | CH₂CONHCH₃ | base | 111-112 |
| 2. | 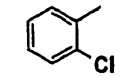 | 1 | CH₂CONHCH₃ | base | 121-122 |
| 3. | 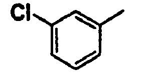 | 1 | CH₂CONHCH₃ | base | 115-116 |
| 4. | 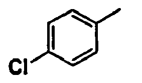 | 1 | CH₂CONHCH₃ | base | 154-155 |
| 5. | 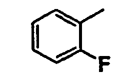 | 1 | CH₂CONHCH₃ | base | 132-133 |
| 6. | 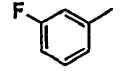 | 1 | CH₂CONHCH₃ | base | 132-133 |
| 7. | 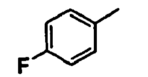 | 1 | CH₂CONHCH₃ | base | 127-128 |
| 8. | 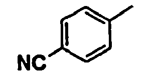 | 1 | CH₂CONHCH₃ | base | 151-152 |
| 9. | 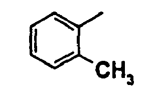 | 1 | CH₂CONHCH₃ | base | 102-103 |
| 10. | 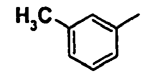 | 1 | CH₂CONHCH₃ | base | 102-103 |
| 11. | 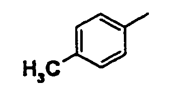 | 1 | CH₂CONHCH₃ | base | 119-120 |
| 12. | 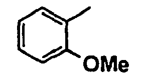 | 1 | CH₂CONHCH₃ | base | 119-120 |
| 13. | 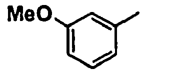 | 1 | CH₂CONHCH₃ | base | 86-87 |
| 14. | 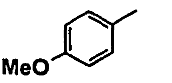 | 1 | CH₂CONHCH₃ | base | 121-122 |
| 15. | 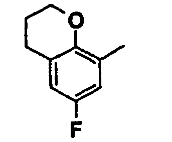 | 1 | CH₂CONHCH₃ | base | 189-190 |
| 16. | 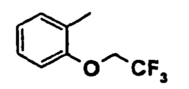 | 1 | CH₂CONHCH₃ | base | 113-114 |
| 17. | 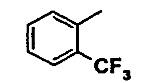 | 1 | CH₂CONHCH₃ | base | 91-93 |
| 18. | 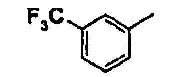 | 1 | CH₂CONHCH₃ | base | 124-125 |
| 19. | | 1 | CH₂CONH₂ | base | M+H=332 |
| 20. | 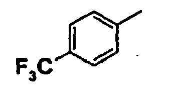 | 1 | CH₂CONHCH₃ | base | 171-172 |
| 21. | 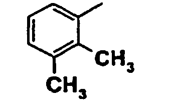 | 1 | CH₂CONHCH₃ | base | 110-111 |
| 22. | 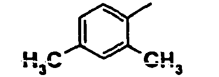 | 1 | CH₂CONHCH₃ | base | 125-126 |
| 23. | 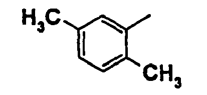 | 1 | CH₂CONHCH₃ | base | 121-122 |
| 24. | 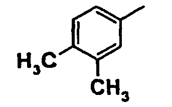 | 1 | CH₂CONHCH₃ | base | 121-122 |
| 25. | 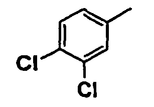 | 1 | CH₂CONHCH₃ | base | 108-109 |
| 26. | 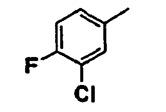 | 1 | CH₂CONHCH₃ | base | 123-124 |
| 27. | 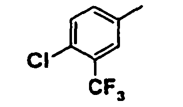 | 1 | CH₂CONHCH₃ | base | 148-149 |
| 28. | 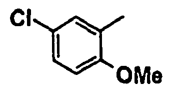 | 1 | CH₂CONHCH₃ | base | 165-166 |
| 29. | 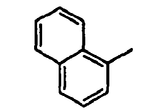 | 1 | CH₂CONHCH₃ | base | 117-118 |
| 30. | 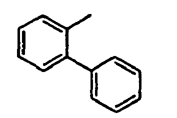 | 1 | CH₂CONHCH₃ | HCl | 172-174 |
| 31. | 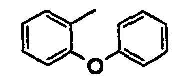 | 1 | CH₂CONHCH₃ | HCl | 103-104 |
| 32. | 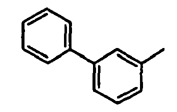 | 1 | CH₂CONHCH₃ | HCl | 199-200 |
| 33. | 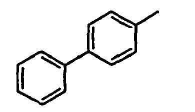 | 1 | CH₂CONHCH₃ | base | 193-194 |
| 34. | 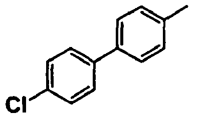 | 1 | CH₂CONHCH₃ | base | 180-181 |
| 35. | 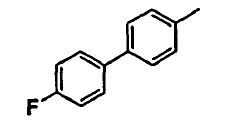 | 1 | CH₂CONHCH₃ | base | 175-176 |
| 36. | 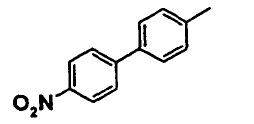 | 1 | CH₂CONHCH₃ | base | 197-198 |
| 37. | 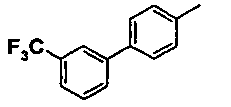 | 1 | CH₂CONHCH₃ | base | 129-130 |
| 38. | 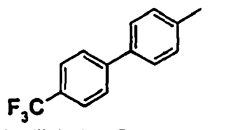 | 1 | CH₂CONHCH₃ | base | 207-208 |
| 39. | 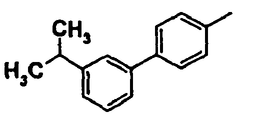 | 1 | CH₂CONHCH₃ | base | 123-124 |
| 40. | 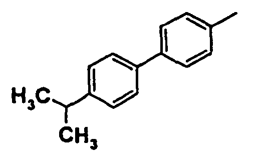 | 1 | CH₂CONHCH₃ | base | 202-203 |
| 41. | 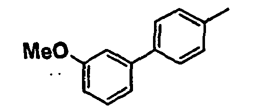 | 1 | CH₂CONHCH₃ | base | 157-158 |
| 42. | 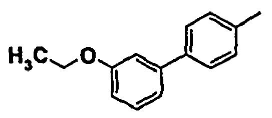 | 1 | CH₂CONHCH₃ | base | 139-140 |
| 43. | 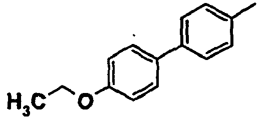 | 1 | CH₂CONHCH₃ | base | 215-216 |
| 44. | 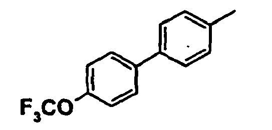 | 1 | CH₂CONHCH₃ | base | 187-189 |
| 45. | | 1 | CH₂CONH₂ | base | 193-195 |
| 46. | 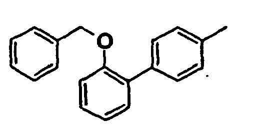 | 1 | CH₂CONHCH₃ | HCl | 199-200 |
| 47. | 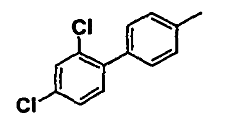 | 1 | CH₂CONHCH₃ | base | 181-182 |
| 48. | 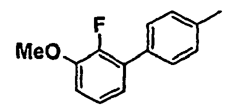 | 1 | CH₂CONHCH₃ | base | 164-165 |
| 49. | 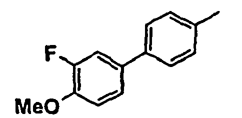 | 1 | CH₂CONHCH₃ | base | 183-184 |
| 50. | 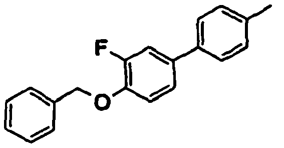 | 1 | CH₂CONHCH₃ | base | 180-181 |
| 51. | 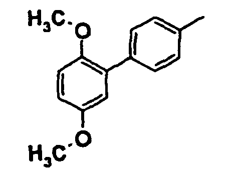 | 1 | CH₂CONHCH₃ | base | M+H=414 |
| 52. | 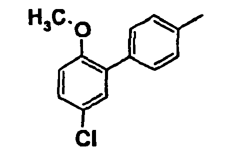 | 1 | CH₂CONHCH₃ | base | 132-133 |
| 53. | 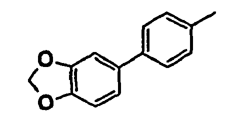 | 1 | CH₂CONHCH₃ | base | 196 |
| 54. | 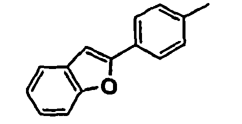 | 1 | CH₂CONHCH₃ | base | 227-228 |
| 55. | 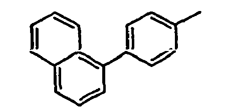 | 1 | CH₂CONHCH₃ | base | 188-189 |
| 56. | 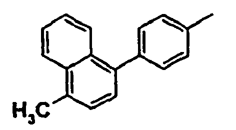 | 1 | CH₂CONHCH₃ | base | 187-189 |
| 57. | 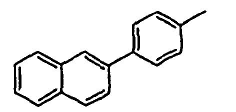 | 1 | CH₂CONHCH₃ | base | 194-195 |
| 58. | 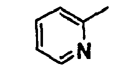 | 1 | CH₂CONHCH₃ | base | 117-118 |
| 59. | 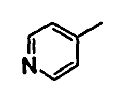 | 1 | CH₂CONHCH₃ | base | 164-165 |
| 60. | 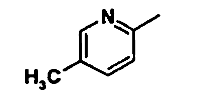 | 1 | CH₂CONHCH₃ | base | 138-140 |
| 61. | | | CH₂CONHCH₂CH₃ | base | 159-161 |
| 62. | | | CH₂CONHCH₂-cyclopropyle | base | 141-143 |
| 63. | 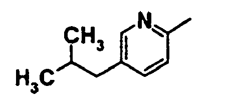 | 1 | CH₂CONHCH₃ | base | 127-129 |
| 64. | 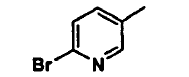 | 1 | CH₂CONHCH₃ | base | 163-165 |
| 65. | 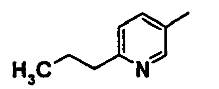 | 1 | CH₂CONHCH₃ | base | 131-132 |
| 66. | 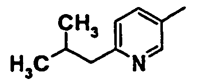 | 1 | CH₂CONHCH₃ | base | 87-89 |
| 67. | 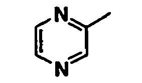 | 1 | CH₂CONHCH₃ | base | 152-154 |
| 68. | | | CH₂CONHCH₂CH₃ | base | 131-133 |
| 69. | 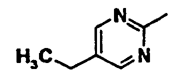 | 1 | CH₂CONHCH₃ | base | 200-204 |
| 70. | | | CH₂CONHCH₂-cyclopropyle | base | 187-189 |
| 71. | 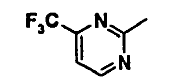 | 1 | CH₂CONHCH₃ | base | 170-172 |
| 72. | | | CH₂CONHCH₂CH₃ | base | 146-148 |
| 73. | 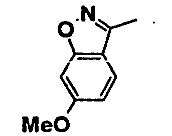 | 1 | CH₂CONHCH₃ | base | 191-192 |
| 74. | 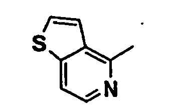 | 1 | CH₂CONHCH₃ | base | 128-129 |
| 75. | 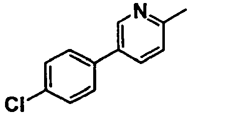 | 1 | CH₂CONHCH₃ | base | 203-206 |
| 76. | 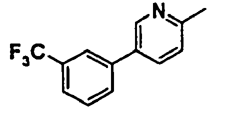 | 1 | CH₂CONHCH₃ | base | 158-160 |
| 77. | | 1 | CH₂CONH₂ | base | 186-188 |
| 78. | 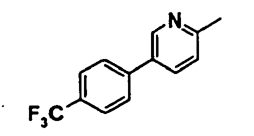 | 1 | CH₂CONH₂ | base | 228-230 |
| 79. | | 1 | CH₂CONHCH₃ | base | 206-209 |
| 80. | | 1 | CH₂CONHCH₂CH₃ | base | 210-212 |
| 81. | | 1 | CH₂CONHCH₂-cyclopropyle | base | 198-200 |
| 82. | | 1 | CH(CH₃)CONHCH3 | base | 198-200 |
| 83. | 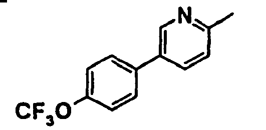 | 1 | CH₂CONHCH₃ | base | 188-190 |
| 84. | 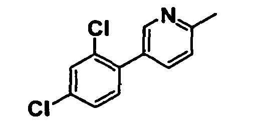 | 1 | CH₂CONHCH₃ | base | 185-187 |
| 85. | 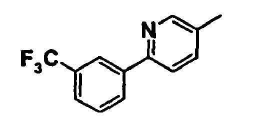 | 1 | CH₂CONHCH₃ | base | 146-150 |
| 86. | | 1 | CH₂CONH₂ | base | 155-157 |
| 87. | 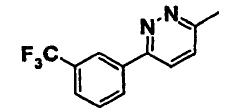 | 1 | CH₂CONHCH₃ | base | 151-153 |
| 88. | 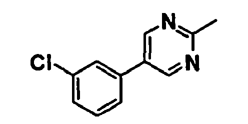 | 1 | CH₂CONHCH₃ | base | 198-202 |
| 89. | 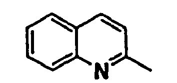 | 1 | CH₂CONHCH₃ | base | 154-158 |
| 90. | 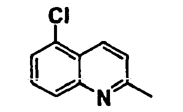 | 1 | CH₂CONH₂ | base | 208-210 |
| 91. | | 1 | CH₂CONHCH₃ | base | 190-191 |
| 92. | 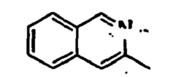 | 1 | CH₂CONH₂ | base | 188-190 |
| 93. | | 1 | CH₂CONHCH₃ | base | 166-168 |
| 94. | 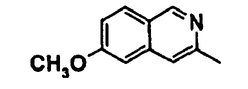 | 1 | CH₂CONHCH₃ | base | 181-183 |
| 95. | 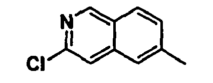 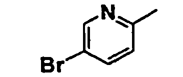 | 1 | CH₂CONH₂ | base | 218-220 |
| 96. | | 1 | CH₂CONHCH₃ | base | 188-190 |
| 97. | | 2 | CH₂CONHCH₃ | base | 156-158 |
| 98. | 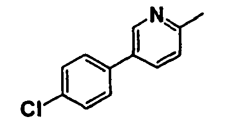 | 2 | CH₂CONHCH₃ | base | 204-206 |
| 99. | 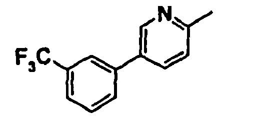 | 2 | CH₂CONHCH₃ | base | 174-176 |
| 100. | 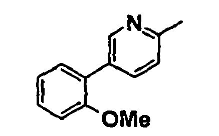 | 2 | CH₂CONHCH₃ | base | 109-111 |
| 101. | 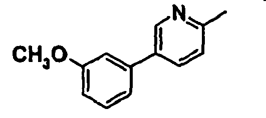 | 2 | CH₂CONHCH₃ | base | 124-126 |
| 102. | 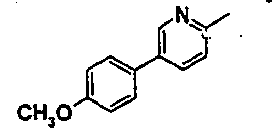 | 2 | CH₂CONHCH₃ | base | 155-157 |
| 103. | 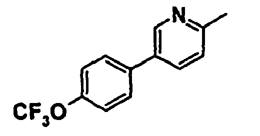 | 2 | CH₂CONHCH₃ | base | 191-193 |
| 104. | 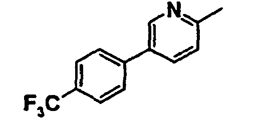 | 2 | CH₂CONHCH₃ | base | M+H=437 |
| 105. | 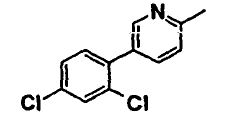 | 2 | CH₂CONHCH₃ | base | 167-170 |
| 106. | 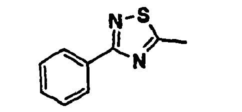 | 2 | CH₂CONHCH₂CH₃ | base | 124-126 |

Les composés de l'invention ont fait l'objet d'essais pharmacologiques permettant de déterminer leur effet inhibiteur de l'enzyme FAAH (Fatty Acid Amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734)*.* Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10 mM (pH 8) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est compté par scintillation liquide.
Dans ces conditions, les composés les plus actifs de l'invention présentent des CI₅₀ (concentration inhibant de 50 % l'activité enzymatique contrôle de la FAAH) comprises entre 0,001 et 1 µM.

Le tableau 2 ci-dessous présente les CI₅₀ de quelques composés selon l'invention.

**Tableau 2**

| Composé N° | CI₅₀ |
|---|---|
| 34 | 0,020 µM |
| 37 | 0,190 µM |
| 43 | 0,044 µM |
| 44 | 0,007 µM |
| 76 | 0,290 µM |
| 83 | 0,012 µM |

Il apparaît donc que les composés selon l'invention ont une activité inhibitrice sur l'enzyme FAAH.

L'activité in vivo des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale en suspension dans du Tween 80 à 0,5 %, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.
Le tableau 3 ci-dessous présente les résultats du test d'analgésie pour quelques composés selon l'invention.

**Tableau 3**

| Composé N° | Réduction du nombre d'étirements (%) |
|---|---|
| 37 | - 57 (a) |
| 44 | - 53 (a) |
| 76 | - 47 (a) |

| | |
|---|---|
| (a) 1 mg/kg p.o. à 2 heures. | |

L'enzyme FAAH (Chemistry and Physics of Lipids, (2000), 108, 107-121) catalyse l'hydrolyse des dérivés endogènes d'amides et d'esters de différents acides gras tels que la N-arachidonoyléthanolamine (anandamide), la *N*-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète ; les douleurs aiguës ou chroniques associées aux maladies inflammatoires : arthrite, arthrite rhumatoïde, ostéoarthrite, spondylite, goutte, vascularite, maladie de Crohn, syndrome du colon irritable ; les douleurs aiguës ou chroniques périphériques; les vertiges; les vomissements ; les nausées en particulier celles consécutives à une chimiothérapie ; les troubles du comportement alimentaire en particulier les anorexies et cachexies de diverses natures ; les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ; les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires ; l'épilepsie ; les troubles du sommeil incluant les apnées du sommeil ; les maladies cardiovasculaires en particulier hypertension, arythmies cardiaques, artériosclérose, crise cardiaque, ischémies cardiaques ; l'ischémie rénale ; les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astro-cytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes) ; les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyo-trophique, amyloses, rejet de greffes, maladies affectant la lignée plasmocytaire ; les maladies allergiques : l'hypersensibilité immédiate ou retardée, rhinites ou conjonctivites allergiques, dermatites de contact ; les maladies infectieuses parasitaires, virales ou bactériennes : SIDA, méningites ; les maladies inflammatoires, notamment les maladies articulaires : arthrite, arthrite rhumatoïde, ostéoarthrite, spondylite, goutte, vascularite, maladie de Crohn, syndrome du colon irritable ; l'ostéoporose ; les affections oculaires : hypertension oculaire, glaucome ; les affections pulmonaires : maladies des voies respiratoires, bronchospasmes, toux, asthme, bronchite chronique, obstruction chronique des voies respiratoires, emphysème ; les maladies gastro-intestinales: syndrome du colon irritable, désordres inflammatoires intestinaux, ulcères, diarrhées ; l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.
Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.
A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la formé galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.
L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 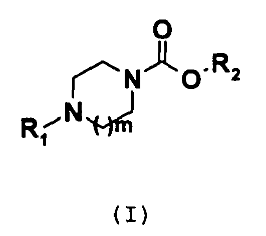 dans laquelle
m représente un nombre entier égal à 1 ou 2 ;
R₁ représente un groupe choisi parmi notamment un phényle, pyridinyle, pyridazinyle, pyrimidinyle, pyrazinyle, triazinyle, oxazolyle, thiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, cinnolinyle, naphthyridinyle, benzofuranyle, dihydrobenzofuranyle, benzothiényle, dihydrobenzothiényle, indolyle, benzoxazolyle, benzisoxazolyle, benzothiazolyle, benzisothiazolyle, benzimidazolyle, indazolyle, pyrrolopyridinyle, furopyridinyle, dihydrofuropyridinyle, thiénopyridinyle, dihydrothiénopyridinyle, imidazopyridinyle, imidazopyrimidinyle, pyrazolopyridinyle, oxazolopyridinyle, isoxazolopyridinyle, thiazolopyridinyle, isothiazolopyridinyle,
ce groupe étant éventuellement substitué par un ou plusieurs groupes R₃ identiques ou différents l'un de l'autre ou par un groupe R₄ ;
R₂ représente un groupe de formule générale CHR₅CONHR₆,
R₃ représente un atome d'halogène ou un groupe hydroxy,cyano, nitro, C₁₋₆-alkyle, C₁₋₆-alcoxy, C₁₋₆-thioalkyle, C₁₋₆-fluoroalkyle, C₁₋₆-fluoroalcoxy, -O-(C₂₋₃-alkylène) -, -O-(C₁₋₃-alkylène)-O-, C₁₋₆-fluorothioalkyle, C₃₋₇-cycloalkyle, C₃₋₇-cycloalkyle-C₁₋₃-alkylène, pipéridinyle, benzyloxy, pipérazinyle, pyrrolidinyle, morpholinyle, phényloxy, NR₇R₈, NHCOR₇, NHSO₂R₇, COR₇, CO₂R₇, CONR₇R₈, SO₂R₇ ou SO₂NR₇R₈, R₄ représente un groupe choisi parmi notamment un phényle, benzofuranyle, naphtyle, pyridinyle, pyrimidinyle, pyridazinyle, pyrazinyle, triazinyle, oxazolyle, thiazolyle, imidazolyle, oxadiazolyle, thiadiazolyle, triazolyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazolpyrimidinyle, benzothiényle, indolyle, benzoxazolyle, benzisoxazolyle, benzothiazolyle, benzisothiazolyle, benzimidazolyle, indazolyle, pyrrolopyridinyle, furopyridinyle, dihydrofuropyridinyle, thiénopyridinyle, dihydrothiénopyridinyle, imidazopyridinyle, imidazopyrimidinyle, pyrazopyridinyle, oxazolopyridinyle, isoxazolopyridinyle, thiazolopyridinyle, isothiazolopyridinyle ;
le ou les groupes R₄ pouvant être substitués par un ou plusieurs groupes R₃ identiques ou différents l'un de l'autre ;
R₅ représente un atome d'hydrogène ou un groupe C₁₋₃-alkyle ;
R₆ représente un atome d'hydrogène ou un groupe C₁₋₆-alkyle, C₃₋₇-cycloalkyle ou C₃₋₇-cycloalkyle-C₁₋₃-alkylène ;
R₇ et R₈ représentent, indépendamment l'un de l'autre, un atome d'hydrogène, un groupe C₁₋₃-alkyle ou un groupe phényle;
à l'état de base, de sel d'addition à un acide, d'hydrate ou de solvat.

2. Composé de formule (I) selon la revendication 1,
**caractérisé en ce que**
m représente un nombre entier égal à 1 ou 2 ;
R₁ représente un groupe choisi parmi notamment un phényle, pyridinyle, pyrimidinyle, pyrazinyle, naphtyle, quinolinyle, isoquinolinyle, benzisoxazolyle, thiénopyridinyle, ce groupe étant éventuellement substitué par un ou plusieurs groupes R₃ identiques ou différents l'un de l'un de l'autre ;
R₂ représente un groupe de formule générale CHR₅CONHR₆ ;
R₃ représente un atome d'halogène ou un groupe cyano, C₁₋₆-alkyle, C₁₋₆-alcoxy, C₁₋₆-fluoroalkyle, C₁₋₆-fluoroalcoxy, -O-(C₂₋₃-alkylène)-, phényloxy ;
R₅ représente un atome d'hydrogène ;
R₆ représente un atome d'hydrogène ou un groupe C₁₋₆-alkyle.

3. Composé de formule (I) selon la revendication 1 ou 2,
**caractérisé en ce que**
m est égal à 1 ;
R₁ représente un groupe choisi parmi notamment un pyridinyle, pyrimidinyle, pyrazinyle, quinolinyle, isoquinolinyle, ce groupe étant éventuellement substitué par un groupe R₃ ;
R₂ représente un groupe de formule générale CHR₅CONHR₆ ;
R₃ représente un atome d'halogène, ou un groupe C₁₋₆-alkyle, C₁₋₆-alcoxy, C₁₋₆-fluoroalkyle ;
R₅ représente un atome d'hydrogène ;
R₆ représente un atome d' hydrogène ou un groupe C₁₋₆-alkyle.

4. Composé de formule (I) selon la revendication 1,
**caractérisé en ce que**
m représente un nombre entier égal à 1 ou 2 ;
R₁ représente un groupe choisi parmi notamment un phényle, pyridinyle, pyridazinyle, pyrimidinyle, thiadiazolyle, ce groupe étant éventuellement substitué par un groupe R₄ ; R₄ représente un groupe choisi parmi notamment un phényle, benzofuranyle, naphtyle ; le groupe R₄ pouvant être substitué par un ou plusieurs groupes R₃ identiques ou différents l'un de l'autre ;
R₂ représente un groupe de formule générale CHR₅CONHR₆ ;
R₃ représente un atome d'halogène ou un groupe nitro, C₁₋₆-alkyle, C₁₋₆-alcoxy, C₁₋₆-fluoroalkyle, C₁₋₆-fluoroalcoxy, -O-(C₁₋₃-alkylène)-O-, benzyloxy ;
R₅ représente un atome d'hydrogène ;
R₆ représente un atome d'hydrogène ou un groupe C₁₋₆-alkyle, ou C₃₋₇-cycloalkyle-C₁₋₃-alkylène.

5. Composé de formule (I) selon la revendication 1 ou 4,
**caractérisé en ce que**
m est égal à 1 ;
R₁ représente un groupe choisi parmi notamment un phényle, pyridinyle, pyridazinyle, pyrimidinyle,
ce groupe étant éventuellement substitué par un groupe R₄ ; R₄ représente un groupe choisi parmi notamment un phényle, benzofuranyle, naphtyle ; le groupe R₄ pouvant être substitué par un ou plusieurs groupes R₃ identiques ou différents l'un de l'autre ;
R₂ représente un groupe de formule générale CHR₅CONHR₆ ;
R₃ représente un atome d'halogène ou un groupe nitro, C₁₋₆-alkyle, C₁₋₆-alcoxy, C₁₋₆-fluoroalkyle, C₁₋₆-fluoroalcoxy, -O-(C₁₋₃-alkylène)-O-, benzyloxy ;
R₅ représente un atome d'hydrogène ;
R₆ représente un atome d'hydrogène ou un groupe C₁₋₆-alkyle.

6. Composé de formule (I) selon la revendication 1 choisi parmi les composés suivants :
4-phénylpipérazine-1-carboxylate de 2-(méthylamino)-2-oxoéthyle ;
4-(2-chlorophényl)pipérazine-1-carboxylate de 2-(méthylamino)-2-oxoéthyle ;
4-(3-chlorophényl)pipérazine-1-carboxylate de 2-(méthylamino)-2-oxoéthyle ;
4-(4-chlorophényl)pipérazine-1-carboxylate de 2-(méthylamino)-2-oxoéthyle ;
4-(2-fluorophényl)pipérazine-1-carboxylate de 2-(méthylamino)-2-oxoéthyle ;
4-(3-fluorophényl)pipérazine-1-carboxylate 2-(méthylamino)-2-oxoéthyle ;
4-(4-fluorophényl)pipérazine-1-carboxylate de 2-(méthylamino)-2-oxoéthyle ;
4-(4-cyanophényl)pipérazine-1-carboxylate de 2-(méthylamino)-2-oxoéthyle ;
4-(2-méthylphényl)pipérazine-1-carboxylate de 2-(méthylamino)-2-oxoéthyle ;
4-(3-méthylphényl)pipérazine-1-carboxylate de 2-(méthylamino)-2-oxoéthyle ;
4-(4-méthylphényl)pipérazine-1-carboxylate de 2-(méthylamino)-2-oxoéthyle ;
4-(2-méthoxyphényl)pipérazine-1-carboxylate de 2-(méthylamino)-2-oxoéthyle ;
4-(3-méthoxyphényl)pipérazine-1-carboxylate de 2-(méthylamino)-2-oxoéthyle ;
4-(4-méthoxyphényl)pipérazine-1-carboxylate de 2-(méthylamino)-2-oxoéthyle ;
4-(6-fluoro-3,4-dihydro-2H-chromèn-8-yl)pipérazine-1-carboxylate de 2-(méthylamino)-2-oxoéthyle ;
4-[2-(2,2,2-trifluoroéthoxy)phényl]pipérazine-1-carboxylate de 2-(méthylamino)-2-oxoéthyle ;
4-[2-(trifluorométhyl)phényl]pipérazine-1-carboxylate de 2-(méthylamino)-2-oxoéthyle ;
4-[3-(trifluorométhyl)phényl]pipérazine-1-carboxylate de 2-(méthylamino)-2-oxoéthyle ;
4-[3-(trifluorométhyl)phényl]pipérazine-1-carboxylate de 2-amino-2-oxoéthyle ;
9-[4-(trifluorométhyl)phényl]pipérazine-1-carboxylate de 2-(méthylamino)-2-oxoéthyle ;
9-(2,3-diméthylphényl)pipérazine-1-carboxylate de 2-(méthylamino)-2-oxoéthyle ;
4-(2,9-diméthylphényl)pipérazine-1-carboxylate de 2-(méthylamino)-2-oxoéthyle ;
4-(2,5-diméthylphényl)pipérazine-1-carboxylate de 2-(méthylamino)-2-oxoéthyle :
4-(3,4-diméthylphényl)pipérazine-1-carboxylate de 2-(méthylamino)-2-oxoéthyle ;
4-(3,4-dichlorophényl)pipérazine-1-carboxylate de 2-(méthylamino)-2-oxoéthyle ;
4-(3-chloro-4-fluorophényl)pipérazine-1-carboxylate de 2-(méthylamino)-2-oxoéthyle ;
4-[4-chloro-3-(trifluorométhyl)phényl]pipérazine-1-carboxylate de 2-(méthylamino)-2-oxoéthyle ;
4-(5-chloro-2-méthoxyphényl)pipérazine-1-carboxylate de 2-(méthylamino)-2-oxoéthyle ;
4-naphthalèn-1-ylpipérazine-1-carboxylate de 2-(méthylamino)-2-oxoéthyle ;
4-biphényl-2-ylpipérazine-1-carboxylate de 2-(méthylamino)-2-oxoéthyle ;
4-(2-phénoxyphényl)pipérazine-1-carboxylate de 2-(méthylamino)-2-oxoéthyle ;
4-biphényl-3-ylpipérazine-1-carboxylate de 2-(méthylamino)-2-oxoéthyle ;
4-biphényl-4-ylpipérazine-1-carboxylate de 2-(méthylamino)-2-oxoéthyle ;
4-(4'-chlorobiphényl-4-yl)pipérazine-l-carboxylate de 2-(méthylamino)-2-oxoéthyle ;
4-(4'-fluorobiphényl-4-yl)pipérazine-1-carboxylate de 2-(méthylamino)-2-oxoéthyle ;
4-(4'-nitrobiphényl-4-yl)pipérazine-1-carboxylate de 2-(méthylamino)-2-oxoéthyle ;
4-[3'-(trifluorométhyl)biphényl-4-yl]pipérazine-1-carboxylate de 2-(méthylamino)-2-oxoéthyle ;
4-[4'-(trifluorométhyl)biphényl-4-yl]pipérazine-1-carboxylate de 2-(méthylamino)-2-oxoéthyle ;
4-[3'-(1-méthyléthyl)biphényl-4-yl]pipérazine-1-carboxylate de 2-(méthylamino)-2-oxoéthyle ;
4-[4'-(1-méthyléthyl)biphényl-4-yl]pipérazine-1-carboxylate de 2-(méthylamino)-2-oxoéthyle ;
4-(3'-méthoxybiphényl-4-yl)pipérazine-1-carboxylate de 2-(méthylamino)-2-oxoéthyle ;
4-(3'-éthoxybiphényl-4-yl)pipérazine-1-carboxylate de 2-(méthylamino)-2-oxoéthyle ;
4-(4'-éthoxybiphényl-4-yl)pipérazine-1-carboxylate de 2-(méthylamino)-2-oxoéthyle ;
4-[4'-(trifluorométhoxy)biphényl-4-yl]pipérazine-1-carboxylate de 2-(méthylamino)-2-oxoéthyle ;
4-[4'-(trifluorométhoxy)biphényl-4-yl]pipérazine-1-carboxylate de 2-amino-2-oxoéthyle ;
4-[2'-(benzyloxy)biphényl-4-yl]pipérazine-1-carboxylate de 2-(méthylamino)-2-oxoéthyle ;
4-(2',4'-dichlorobiphényl-4-yl)pipérazine-1-carboxylate de 2-(méthylamino)-2-oxoéthyle ;
4-(2'-fluoro-3'-méthoxybiphényl-4-yl)pipérazine-1-carboxylate de 2-(méthylamino)-2-oxoéthyle ;
4-(3'-fluoro-4'-méthoxybiphényl-4-yl)pipérazine-1-carboxylate de 2-(méthylamino)-2-oxoéthyle ;
4-[4'-(benzyloxy)-3'-fluorobiphényl-4-yl]pipérazine-1-carboxylate de 2-(méthylamino)-2-oxoéthyle ;
4-(2',5'-diméthoxybiphényl-4-yl)pipérazine-1-carboxylate de 2-(méthylamino)-2-oxoéthyle ;
4-(5'-chloro-2'-méthoxybiphényl-4-yl)pipérazine-1-carboxylate de 2-(méthylamino)-2-oxoéthyle ;
9-[4-(1,3-benzodioxol-5-yl)phényl]pipérazine-1-carboxylate de 2-(méthylamino)-2-oxoéthyle ;
4-[4-(1-benzofuran-2-yl)phényl]pipérazine-1-carboxylate de 2-(méthylamino)-2-oxoéthyle ;
4-(4-naphthalèn-1-ylphényl)pipérazine-1-carboxylate de 2-(méthylamino)-2-oxoéthyle ;
4-[4-(4-méthylnaphthalèn-1-yl)phényl]pipérazine-1-carboxylate de 2-(méthylamino)-2-oxoéthyle ;
4-(4-naphthalèn-2-ylphényl)pipérazine-1-carboxylate de 2-(méthylamino)-2-oxoéthyle ;
4-pyridin-2-ylpipérazine-1-carboxylate de 2-(méthylamino)-2-oxoéthyle ;
4-pyridin-4-ylpipérazine-1-carboxylate de 2-(méthylamino)-2-oxoéthyle ;
4-(5-méthylpyridin-2-yl)pipérazine-1-carboxylate de 2-(méthylamino)-2-oxoéthyle;
4-(5-méthylpyridin-2-yl)pipérazine-1-carboxylate de 2-(éthylamino)-2-oxoéthyle;
4-(5-méthylpyridin-2-yl)pipérazine-1-carboxylate de 2-[(cyclopropylméthyl)amino]-2-oxoéthyle;
4-[5-(2-méthylpropyl)pyridin-2-yl]pipérazine-1-carboxylate de 2-(méthylamino)-2-oxoéthyle;
4-(6-bromopyridin-3-yl)pipérazine-1-carboxylate de 2-(méthylamino)-2-oxoéthyle;
4-(6-propylpyridin-3-yl)pipérazine-1-carboxylate de 2-(méthylamino)-2-oxoéthyle;
4-[6-(2-méthylpropyl)pyridin-3-yl]pipérazine-1-carboxylate de 2-(méthylamino)-2-oxoéthyle;
4-pyrazin-2-ylpipérazine-1-carboxylate de 2-(méthylamino)-2-oxoéthyle;
4-pyrazin-2-ylpipérazine-1-carboxylate de 2-(éthylamino)-2-oxoéthyle;
4-(5-éthylpyrimidin-2-yl)pipérazine-1-carboxylate de 2-(méthylamino)-2-oxoéthyle;
4-(5-éthylpyrimidin-2-yl)pipérazine-1-carboxylate de 2-[(cyclopropylméthyl)amino]-2-oxoéthyle;
4-[4-(trifluorométhyl)pyrimidin-2-yl]pipérazine-1-carboxylate de 2-(méthylamino)-2-oxoéthyle;
4-[4-(trifluorométhyl)pyrimidin-2-yl]pipérazine-1-carboxylate de 2-(éthylamino)-2-oxoéthyle;
4-(6-méthoxy-1,2-benzisoxazol-3-yl)pipérazine-1-carboxylate de 2-(méthylamino)-2-oxoéthyle;
4-thiéno[3,2-*c*]pyridin-4-ylpipérazine-1-carboxylate de 2-(méthylamino)-2-oxoéthyle;
4-[5-(4-chlorophényl)pyridin-2-yl]pipérazine-1-carboxylate de 2-(méthylamino)-2-oxoéthyle;
4-{5-[3-(trifluorométhyl)phényl]pyridin-2-yl}pipérazine-1-carboxylate de 2-(méthylamino)-2-oxoéthyle;
4-{5-[3-(trifluorométhyl)phényl]pyridin-2-yl}pipérazine-1-carboxylate de 2-amino-2-oxoéthyle;
4-{5-[4-(trifluorométhyl)phényl]pyridin-2-yl}pipérazine-1-carboxylate de 2-amino-2-oxoéthyle;
4-{5-[4-(trifluorométhyl)phényl]pyridin-2-yl}pipérazine-1-carboxylate de 2-(méthylamino)-2-oxoéthyle;
4-{5-[4-(trifluorométhyl)phényl]pyridin-2-yl}pipérazine-1-carboxylate de 2-(éthylamino)-2-oxoéthyle;
4-{5-[4-(trifluorométhyl)phényl]pyridin-2-yl}pipérazine-1-carboxylate de 2-[(cyclopropylméthyl)amino]-2-oxoéthyle;
4-{5-[4-(trifluorométhyl)phényl]pyridin-2-yl}pipérazine-1-carboxylate de 1-méthyl-2-(méthylamino)-2-oxoéthyle;
4-{5-[4-(trifluorométhoxy)phényl]pyridin-2-yl}pipérazine-1-carboxylate de 2-(méthylamino)-2-oxoéthyle;
4-[5-(2,4-dichlorophényl)pyridin-2-yl]pipérazine-1-carboxylate de 2-(méthylamino)-2-oxoéthyle;
4-{6-[3-(trifluorométhyl)phényl]pyridin-3-yl}pipérazine-1-carboxylate de 2-(méthylamino)-2-oxoéthyle;
4-(6-[3-(trifluorométhyl)phényl]pyridin-3-yl)pipérazine-1-carboxylate de 2-amino-2-oxoéthyle;
4-{6-[3-(trifluorométhyl)phényl]pyridazin-3-yl}pipérazine-1-carboxylate de 2-(méthylamino)-2-oxoéthyle;
4-[5-(3-chlorophényl)pyrimidin-2-yl]pipérazine-1-carboxylate de 2-(méthylamino)-2-oxoéthyle;
4-quinolin-2-ylpipérazine-1-carboxylate de 2-(méthylamino)-2-oxoéthyle;
4-(5-chloroquinolin-2-yl)pipérazine-1-carboxylate de 2-amino-2-oxoéthyle;
4-(5-chloroquinolin-2-yl)pipérazine-1-carboxylate de 2-(méthylamino)-2-oxoéthyle;
4-isoquinolin-3-ylpipérazine-1-carboxylate de 2-amino-2-oxoéthyle;
4-isoquinolin-3-ylpipérazine-1-carboxylate de 2-(méthylamino)-2-oxoéthyle;
4-(6-méthoxyisoquinolin-3-yl)pipérazine-1-carboxylate de 2-(méthylamino)-2-oxoéthyle;
9-(3-chloroisoquinolin-6-yl)pipérazine-1-carboxylate de 2-amino-2-oxoéthyle;
4-(3-chloroisoquinolin-6-yl)pipérazine-1-carboxylate de 2-(méthylamino)-2-oxoéthyle;
4-(5-bromopyridin-2-yl)-1,9-diazépane-1-carboxylate de 2-(méthylamino)-2-oxoéthyle;
4-[5-(4-chlorophényl)pyridin-2-yl]-1,4-diazépane-1-carboxylate de 2-(méthylamino)-2-oxoéthyle;
4-{5-[3-(trifluorométhyl)phényl]pyridin-2-yl}-1,4-diazépane-1-carboxylate de 2-(méthylamino)-2-oxoéthyle;
4-[5-(2-méthoxyphényl)pyridin-2-yl]-1,4-diazépane-1-carboxylate de 2-(méthylamino)-2-oxoéthyle;
4-[5-(3-méthoxyphényl)pyridin-2-yl]-1,4-diazépane-1-carboxylate de 2-(méthylamino)-2-oxoéthyle;
4-[5-(4-méthoxyphényl)pyridin-2-yl]-1,4-diazépane-1-carboxylate de 2-(méthylamino)-2-oxoéthyle;
4-{5-[4-(trifluorométhoxy)phényl]pyridin-2-yl}-1,4-diazépane-1-carboxylate de 2-(méthylamino)-2-oxoéthyle;
4-{5-[4-(trifluorométhyl)phényl]pyridin-2-yl}-1,4-diazépane-1-carboxylate de 2-(méthylamino)-2-oxoéthyle;
4-[5-(2,4-dichlorophényl)pyridin-2-yl]-1,4-diazépane-1-carboxylate de 2-(méthylamino)-2-oxoéthyle;
4-(3-phényl-1,2,4-thiadiazol-5-yl)-1,9-diazépane-1-carboxylate de 2-(éthylamino)-2-oxoéthyle.

7. Procédé de préparation d'un composé de formule (I) selon l'une quelconque des revendications 1 à 6, comprenant l'étape consistant à faire réagir une amine de formule générale (II), 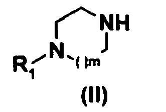 dans laquelle R₁ et m sont tels que définis dans la formule générale (I) selon la revendication 1,
avec un carbonate de formule générale (III), 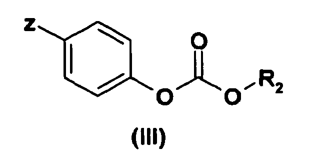 dans laquelle Z représente un atome d'hydrogène ou un groupement nitro et R₂ est tel que défini dans la formule générale (I) selon la revendication 1.

8. Procédé de préparation d'un composé de formule (I) selon l'une quelconque des revendications 1 à 6, comprenant l'étape consistant à transformer le carbamate-ester de formule générale (Ia) 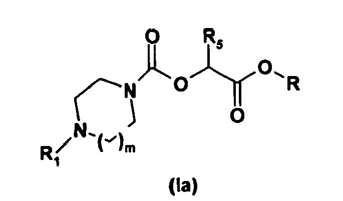 dans laquelle m, R₁ et R₅ sont tels que définis dans la formule générale (I) selon la revendication 1 et R représente un groupe méthyle ou éthyle,
par aminolyse au moyen d'une amine de formule générale R₆NH₂ dans laquelle R₆ est tel que défini dans la formule générale (I) selon la revendication 1.

9. Composé répondant à la formule générale (III), qui est le 4-nitrophényle carbonate de 2-(méthylamino)-2-oxoéthyle.

10. Composé répondant à la formule générale (Ia), 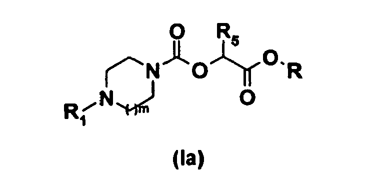 dans laquelle m, R₁ et R₅ sont tels que définis dans la formule générale (I) selon la revendication 1 et R représente un groupe méthyle ou éthyle.

11. Composé de formule (I) selon l'une quelconque des revendications 1 à 6, à l'état de base, de sel d'addition à un acide, d'hydrate ou de solvat pharmaceutiquement acceptable, pour son utilisation comme médicament.

12. Composition pharmaceutique contenant au moins un composé de formule (I) selon l'une quelconque des revendications 1 à 6, à l'état de base, de sel d'addition à un acide, d'hydrate ou de solvat pharmaceutiquement acceptable et éventuellement un ou plusieurs excipients pharmaceutiquement acceptables.

13. Utilisation d'un composé de formule (I) selon l'une quelconque des revendications 1 à 6, à l'état de base, de sel d'addition à un acide, d'hydrate ou de solvat pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

14. Utilisation d'un composé de formule (I) selon l'une quelconque des revendications 1 à 6, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

15. Utilisation selon la revendication 14 **caractérisée en ce que** les douleurs aiguës ou chroniques sont de type neurogène, périphériques ou associées aux maladies inflammatoires.

16. Utilisation selon la revendication 14 **caractérisée en ce que** les troubles du comportement alimentaire correspondent aux anorexies et cachexies.

17. Utilisation selon la revendication 14 **caractérisée en ce que** les pathologies neurologiques et psychiatriques sont choisies parmi les pathologies suivantes : tremblements, dyskinésies, dystonies, spasticité, comportements compulsifs et obsessionnels, syndrome de Tourette, dépression, anxiété, troubles de l'humeur, psychoses.

18. Utilisation selon la revendication 14 **caractérisée en ce que** les maladies neuro-dégénératives aiguës et chroniques sont choisies parmi les maladies suivantes : maladie de Parkinson, maladie d'Alzheimer, démence sénile, chorée de Huntington, lésions liées à l'ischémie cérébrale et aux traumatismes crâniens et médullaires.

19. Utilisation selon la revendication 14 **caractérisée en ce que** les maladies cardiovasculaires sont choisies parmi les maladies suivantes : hypertension, arythmies cardiaques, artériosclérose, crise cardiaque, ischémies cardiaques.

20. Utilisation selon la revendication 14 **caractérisée en ce que** les cancers sont choisis parmi les tumeurs bénignes de la peau, les papillomes et tumeurs cérébrales, les tumeurs de la prostate.

21. Utilisation selon la revendication 14 **caractérisée en ce que** les désordres du système immunitaire sont les maladies auto-immunes.

22. Utilisation selon la revendication 14 **caractérisée en ce que** les maladies allergiques sont choisies parmi : l'hypersensibilité immédiate ou retardée, les rhinites ou conjonctivites allergiques, les dermatites de contact.

23. Utilisation selon la revendication 14 **caractérisée en ce que** les maladies inflammatoires sont les maladies articulaires.

24. Utilisation selon la revendication 14 **caractérisée en ce que** les affections pulmonaires sont choisies parmi : les maladies des voies respiratoires, les bronchospasmes, la toux, l'asthme, la bronchite chronique, l'obstruction chronique des voies respiratoires, l'emphysème.

25. Utilisation selon la revendication 14 **caractérisée en ce que** les maladies gastro-intestinales sont choisies parmi : le syndrome du colon irritable, les désordres inflammatoires intestinaux, les ulcères, les diarrhées.

## Claims

1. Compound corresponding to formula (I) 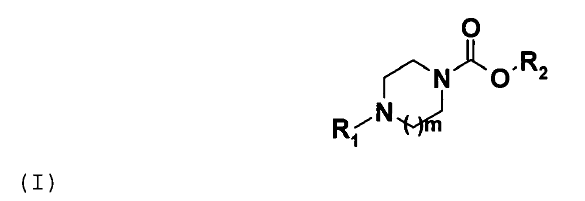 in which
m represents an integer equal to 1 or 2;
R₁ represents a group chosen especially from a phenyl, pyridyl, pyridazinyl, pyrimidinyl, pyrazinyl, triazinyl, oxazolyl, thiazolyl, imidazolyl, oxadiazolyl, thiadiazolyl, triazolyl, naphthyl, quinolyl, tetrahydroquinolyl, isoquinolyl, tetrahydroisoquinolyl, phthalazinyl, quinazolinyl, quinoxalinyl, cinnolinyl, naphthyridinyl, benzofuryl, dihydrobenzofuryl, benzothienyl, dihydrobenzothienyl, indolyl, benzoxazolyl, benzisoxazolyl, benzothiazolyl, benzisothiazolyl, benzimidazolyl, indazolyl, pyrrolopyridyl, furopyridyl, dihydrofuropyridyl, thienopyridyl, dihydrothienopyridyl, imidazopyridyl, imidazopyrimidinyl, pyrazolo-pyridyl, oxazolopyridyl, isoxazolopyridyl, thiazolopyridyl or isothiazolopyridyl,
this group being optionally substituted with one or more groups R₃, which may be identical or different, or with a group R₄;
R₂ represents a group of general formula CHR₅CONHR₆,
R₃ represents a halogen atom or a hydroxyl, cyano, nitro, C₁₋₆-alkyl, C₁₋₆-alkoxy, C₁₋₆-thioalkyl, C₁₋₆-fluoroalkyl, C₁₋₆-fluoroalkoxy, -O-(C₂₋₃-alkylene)-, -0-(C₁₋₃-alkylene)-O-, C₁₋₆-fluorothioalkyl, C₃₋₇-cycloalkyl, C₃₋₇-cycloalkyl-C₁₋₃-alkylene, piperidyl, benzyloxy, piperazinyl, pyrrolidinyl, morpholinyl, phenyloxy, NR₇R₈, NHCOR₇, NHSO₂R₇, COR₇, CO₂R₇, CONR₇R₈, SO₂R₇ or SO₂NR₇R₈ group,
R₄ represents a group chosen especially from a phenyl, benzofuryl, naphthyl, pyridyl, pyrimidinyl, pyridazinyl, pyrazinyl, triazinyl, oxazolyl, thiazolyl, imidazolyl, oxadiazolyl, thiadiazolyl, triazolyl, quinolyl, tetrahydroquinolyl, isoquinolyl, tetrahydroisoquinolyl, phthalazinyl, quinazolinyl, quinoxalinyl, naphthyridinyl, cinnolinyl, imidazolpyrimidinyl, benzothienyl, indolyl, benzoxazolyl, benzisoxazolyl, benzothiazolyl, benzisothiazolyl, benzimidazolyl, indazolyl, pyrrolopyridyl, furopyridyl, dihydrofuropyridyl, thienopyridyl, dihydrothienopyridyl, imidazopyridyl, imidazopyrimidinyl, pyrazopyridyl, oxazolopyridyl, isoxazolopyridyl, thiazolopyridyl and isothiazolopyridyl;
the group(s) R₄ possibly being substituted with one or more groups R₃, which may be identical or different;
R₅ represents a hydrogen atom or a C₁₋₃-alkyl group;
R₆ represents a hydrogen atom or a C₁₋₆-alkyl, C₃₋₇-cycloalkyl or C₃₋₇-cycloalkyl-C₁₋₃-alkylene group;
R₇ and R₈ represent, independently of each other, a hydrogen atom, a C₁₋₃-alkyl group or a phenyl group;
in the form of base, of addition salt with an acid, of hydrate or of solvate.

2. Compound of formula (I) according to Claim 1,
**characterized in that**
m represents an integer equal to 1 or 2;
R₁ represents a group chosen especially from a phenyl, pyridyl, pyrimidinyl, pyrazinyl, naphthyl, quinolyl, isoquinolyl, benzisoxazolyl and thienopyridyl, this group being optionally substituted with one or more groups R₃, which may be identical or different;
R₂ represents a group of general formula CHR₅CONHR₆;
R₃ represents a halogen atom or a cyano, C₁₋₆-alkyl, C₁₋₆-alkoxy, C₁₋₆-fluoroalkyl, C₁₋₆-fluoroalkoxy, -O- (C₂₋₃-alkylene)- or phenyloxy group;
R₅ represents a hydrogen atom;
R₆ represents a hydrogen atom or a C₁₋₆-alkyl group.

3. Compound of formula (I) according to Claim 1 or 2,
**characterized in that**
m is equal to 1;
R₁ represents a group chosen especially from a pyridyl, pyrimidinyl, pyrazinyl, quinolyl and isoquinolyl, this group being optionally substituted with a group R₃;
R₂ represents a group of general formula CHR₅CONHR_{6;}
R₃ represents a halogen atom, or a C₁₋₆-alkyl, C₁₋₆-alkoxy or C₁₋₆-fluoroalkyl group;
R₅ represents a hydrogen atom;
R₆ represents a hydrogen atom or a C₁₋₆-alkyl group.

4. Compound of formula (I) according to Claim 1,
**characterized in that**
m represents an integer equal to 1 or 2;
R₁ represents a group chosen especially from a phenyl, pyridyl, pyridazinyl, pyrimidinyl and thiadiazolyl, this group being optionally substituted with a group R₄; R₄ represents a group chosen especially from a phenyl, benzofuryl and naphthyl; the group R₄ possibly being substituted with one or more groups R₃, which may be identical or different;
R₂ represents a group of general formula CHR₅CONHR_{6;}
R₃ represents a halogen atom or a nitro, C₁₋₆-alkyl, C₁₋₆-alkoxy, C₁₋₆-fluoroalkyl, C₁₋₆-fluoroalkoxy, -O-(C₁₋₃-alkylene)-O- or benzyloxy group;
R₅ represents a hydrogen atom;
R₆ represents a hydrogen atom or a C₁₋₆-alkyl or C₃₋₇-cycloalkyl-C₁₋₃-alkylene group.

5. Compound of formula (I) according to Claim 1 or 4,
**characterized in that**
m is equal to 1;
R₁ represents a group chosen especially from a phenyl, pyridyl, pyridazinyl and pyrimidinyl,
this group being optionally substituted with a group R₄; R₄ represents a group chosen especially from a phenyl, benzofuryl and naphthyl; the group R₄ possibly being substituted with one or more groups R₃, which may be identical or different;
R₂ represents a group of general formula CHR₅CONHR₆;
R₃ represents a halogen atom or a nitro, C₁₋₆-alkyl, C₁₋₆-alkoxy, C₁₋₆-fluoroalkyl, C₁₋₆-fluoroalkoxy, -O-(C₁₋₃-alkylene)-O- or benzyloxy group;
R₅ represents a hydrogen atom;
R₆ represents a hydrogen atom or a C₁₋₆-alkyl group.

6. Compound of formula (I) according to Claim 1, chosen from the following compounds:
2-(methylamino)-2-oxoethyl 4-phenyl-1-piperazinecarboxylate;
2-(methylamino)-2-oxoethyl 4-(2-chlorophenyl)-1-piperazinecarboxylate;
2-(methylamino)-2-oxoethyl 4-(3-chlorophenyl)-1-piperazinecarboxylate;
2-(methylamino)-2-oxoethyl 4-(4-chlorophenyl)-1-piperazinecarboxylate;
2-(methylamino)-2-oxoethyl 4-(2-fluorophenyl)-1-piperazinecarboxylate;
2-(methylamino)-2-oxoethyl 4-(3-fluorophenyl)-1-piperazinecarboxylate;
2-(methylamino)-2-oxoethyl 4-(4-fluorophenyl)-1-piperazinecarboxylate;
2-(methylamino)-2-oxoethyl 4-(4-cyanophenyl)-1-piperazinecarboxylate;
2-(methylamino)-2-oxoethyl 4-(2-methylphenyl)-1-piperazinecarboxylate;
2-(methylamino)-2-oxoethyl 4-(3-methylphenyl)-1-piperazinecarboxylate;
2-(methylamino)-2-oxoethyl 4-(4-methylphenyl)-1-piperazinecarboxylate;
2-(methylamino)-2-oxoethyl 4-(2-methoxyphenyl)-1-piperazinecarboxylate;
2-(methylamino)-2-oxoethyl 4-(3-methoxyphenyl)-1-piperazinecarboxylate;
2-(methylamino)-2-oxoethyl 4-(4-methoxyphenyl)-1-piperazinecarboxylate;
2-(methylamino)-2-oxoethyl 4-(6-fluoro-3,4-dihydro-2H-chromen-8-yl)-1-piperazinecarboxylate;
2-(methylamino)-2-oxoethyl 4-[2-(2,2,2-trifluoro-ethoxy)phenyl]-1-piperazinecarboxylate;
2-(methylamino)-2-oxoethyl 4-[2-(trifluoromethyl)-phenyl]-1-piperazinecarboxylate;
2-(methylamino)-2-oxoethyl 4-[3-(trifluoromethyl)-phenyl]-1-piperazinecarboxylate;
2-amino-2-oxoethyl 4-[3-(trifluoromethyl)phenyl]-1-piperazinecarboxylate;
2-(methylamino)-2-oxoethyl 4-[4-(trifluoromethyl)-phenyl]-1-piperazinecarboxylate;
2-(methylamino)-2-oxoethyl 4-(2,3-dimethylphenyl)-1-piperazinecarboxylate;
2-(methylamino)-2-oxoethyl 4-(2,4-dimethylphenyl)-1-piperazinecarboxylate;
2-(methylamino)-2-oxoethyl 4-(2,5-dimethylphenyl)-1-piperazinecarboxylate;
2-(methylamino)-2-oxoethyl 4-(3,4-dimethylphenyl)-1-piperazinecarboxylate;
2-(methylamino)-2-oxoethyl 4-(3,4-dichlorophenyl)-1-piperazinecarboxylate;
2-(methylamino)-2-oxoethyl 4-(3-chloro-4-fluorophenyl)-1-piperazinecarboxylate;
2-(methylamino)-2-oxoethyl 4-[4-chloro-3-(trifluoromethyl) phenyl]-1-piperazinecarboxylate;
2-(methylamino)-2-oxoethyl 4-(5-chloro-2-methoxyphenyl)-1-piperazinecarboxylate;
2-(methylamino)-2-oxoethyl 4-naphth-1-yl-1-piperazinecarboxylate;
2-(methylamino)-2-oxoethyl 4-biphenyl-2-yl-1-piperazinecarboxylate;
2-(methylamino)-2-oxoethyl 4-(2-phenoxyphenyl)-1-piperazinecarboxylate;
2-(methylamino)-2-oxoethyl 4-biphenyl-3-yl-1-piperazinecarboxylate;
2-(methylamino)-2-oxoethyl 4-biphenyl-4-yl-1-piperazinecarboxylate;
2-(methylamino)-2-oxoethyl 4-(4'-chlorobiphenyl-4-yl)-1-piperazinecarboxylate;
2-(methylamino)-2-oxoethyl 4-(4'-fluorobiphenyl-4-yl)-1-piperazinecarboxylate;
2-(methylamino)-2-oxoethyl 4-(4'-nitrobiphenyl-4-yl)-1-piperazinecarboxylate;
2-(methylamino)-2-oxoethyl 4-[3'-(trifluoromethyl)-biphenyl-4-yl]-1-piperazinecarboxylate;
2-(methylamino)-2-oxoethyl 4-[4'-(trifluoromethyl)-biphenyl-4-yl]-1-piperazinecarboxylate;
2-(methylamino)-2-oxoethyl 4-[3'-(1-methylethyl)-biphenyl-4-yl]-1-piperazinecarboxylate;
2-(methylamino)-2-oxoethyl 4-[4'-(1-methylethyl)-biphenyl-4-yl]-1-piperazinecarboxylate;
2-(methylamino)-2-oxoethyl 4-(3'-methoxybiphenyl-4-yl)-1-piperazinecarboxylate;
2-(methylamino)-2-oxoethyl 4-(3'-ethoxybiphenyl-4-yl)-1-piperazinecarboxylate;
2-(methylamino)-2-oxoethyl 4-(4'-ethoxybiphenyl-4-yl)-1-piperazinecarboxylate;
2-(methylamino)-2-oxoethyl 4-[4'-(trifluoromethoxy)-biphenyl-4-yl]-1-piperazinecarboxylate;
2-amino-2-oxoethyl 4-[4'-(trifluoromethoxy)biphenyl-4-yl]-1-piperazinecarboxylate;
2-(methylamino)-2-oxoethyl 4-[2'-(benzyloxy)biphenyl-4-yl]-1-piperazinecarboxylate;
2-(methylamino)-2-oxoethyl 4-(2',4'-dichlorobiphenyl-4-yl)-1-piperazinecarboxylate;
2-(methylamino)-2-oxoethyl 4-(2'-fluoro-3'-methoxy-biphenyl-4-yl)-1-piperazinecarboxylate;
2-(methylamino)-2-oxoethyl 4-(3'-fluoro-4'-methoxy-biphenyl-4-yl)-1-piperazinecarboxylate;
2-(methylamino)-2-oxoethyl 4-[4'-(benzyloxy)-3'-fluoro-biphenyl-4-yl]-1-piperazinecarboxylate;
2-(methylamino)-2-oxoethyl 4-(2',5'-dimethoxybiphenyl-4-yl)-1-piperazinecarboxylate;
2-(methylamino)-2-oxoethyl 4-(5'-chloro-2'-methoxy-biphenyl-4-yl)-1-piperazinecarboxylate;
2-(methylamino)-2-oxoethyl 4-[4-(1,3-benzodioxol-5-yl)-phenyl]-1-piperazinecarboxylate;
2-(methylamino)-2-oxoethyl 4-[4-(1-benzofuran-2-yl)-phenyl]-1-piperazinecarboxylate;
2-(methylamino)-2-oxoethyl 4-(4-naphth-1-ylphenyl)-1-piperazinecarboxylate;
2-(methylamino)-2-oxoethyl 4-[4-(4-methyl-1-naphthyl)-phenyl]-1-piperazinecarboxylate;
2-(methylamino)-2-oxoethyl 4-(4-naphth-2-ylphenyl)-1-piperazinecarboxylate;
2-(methylamino)-2-oxoethyl 4-pyrid-2-yl-1-piperazinecarboxylate;
2-(methylamino)-2-oxoethyl 4-pyrid-4-yl-1-piperazinecarboxylate;
2-(methylamino)-2-oxoethyl 4-(5-methyl-2-pyridyl)-1-piperazinecarboxylate;
2-(ethylamino)-2-oxoethyl 4-(5-methyl-2-pyridyl)-1-piperazinecarboxylate;
2-[(cyclopropylmethyl)amino]-2-oxoethyl 4-(5-methyl-2-pyridyl)-1-piperazinecarboxylate;
2-(methylamino)-2-oxoethyl 4-[5-(2-methylpropyl)-2-pyridyl]-1-piperazinecarboxylate;
2-(methylamino)-2-oxoethyl 4-(6-bromo-3-pyridyl)-1-piperazinecarboxylate;
2-(methylamino)-2-oxoethyl 4-(6-propyl-3-pyridyl)-1-piperazinecarboxylate;
2-(methylamino)-2-oxoethyl 4-[6-(2-methylpropyl)-3-pyridyl]-1-piperazinecarboxylate;
2-(methylamino)-2-oxoethyl 4-pyrazin-2-yl-1-piperazinecarboxylate;
2-(ethylamino)-2-oxoethyl 4-pyrazin-2-yl-1-piperazinecarboxylate;
2-(methylamino)-2-oxoethyl 4-(5-ethyl-2-pyrimidinyl)-1-piperazinecarboxylate;
2-[(cyclopropylmethyl)amino]-2-oxoethyl 4-(5-ethyl-2-pyrimidinyl)-1-piperazinecarboxylate;
2-(methylamino)-2-oxoethyl 4-[4-(trifluoromethyl)-2-pyrimidinyl]-1-piperazinecarboxylate;
2-(ethylamino)-2-oxoethyl 4-[4-(trifluoromethyl)-2-pyrimidinyl]-1-piperazinecarboxylate;
2-(methylamino)-2-oxoethyl 4-(6-methoxy-1,2-benzisoxazol-3-yl)-1-piperazinecarboxylate;
2-(methylamino)-2-oxoethyl 4-thieno[3,2-*c*]-4-pyridyl-1-piperazinecarboxylate;
2-(methylamino)-2-oxoethyl 4-[5-(4-chlorophenyl)-2-pyridyl]-1-piperazinecarboxylate;
2-(methylamino)-2-oxoethyl 4-{5-[3-(trifluoromethyl)-phenyl]-2-pyridyl}-1-piperazinecarboxylate;
2-amino-2-oxoethyl 4-{5-[3-(trifluoromethyl)phenyl]-2-pyridyl}-1-piperazinecarboxylate;
2-amino-2-oxoethyl 4-{5-[4-(trifluoromethyl)phenyl]-2-pyridyl}-1-piperazinecarboxylate;
2-(methylamino)-2-oxoethyl 4-{5-[4-(trifluoromethyl)-phenyl]-2-pyridyl}-1-piperazinecarboxylate;
2-(ethylamino)-2-oxoethyl 4-{5-[4-(trifluoromethyl)-phenyl]-2-pyridyl}-1-piperazinecarboxylate;
2-[(cyclopropylmethyl)amino]-2-oxoethyl 4-{5-[4-(trifluoromethyl)phenyl]-2-pyridyl}-1-piperazinecarboxylate;
1-methyl-2-(methylamino)-2-oxoethyl 4-{5-[4-(trifluoromethyl)phenyl]-2-pyridyl}-1-piperazinecarboxylate;
2-(methylamino)-2-oxoethyl 4-{5-[4-(trifluoromethoxy)phenyl]-2-pyridyl}-1-piperazinecarboxylate;
2-(methylamino)-2-oxoethyl 4-[5-(2,4-dichlorophenyl)-2-pyridyl]-1-piperazinecarboxylate;
2-(methylamino)-2-oxoethyl 4-{6-[3-(trifluoromethyl)-phenyl]-3-pyridyl}-1-piperazinecarboxylate;
2-amino-2-oxoethyl 4-{6-[3-(trifluoromethyl)phenyl]-3-pyridyl}-1-piperazinecarboxylate;
2-(methylamino)-2-oxoethyl 4-{6-[3-(trifluoromethyl)-phenyl]-3-pyridazinyl}-1-piperazinecarboxylate;
2-(methylamino)-2-oxoethyl 4-[5-(3-chlorophenyl)-2-pyrimidinyl]-1-piperazinecarboxylate;
2-(methylamino)-2-oxoethyl 4-quinol-2-yl-1-piperazinecarboxylate;
2-amino-2-oxoethyl 4-(5-chloro-2-quinolyl)-1-piperazinecarboxylate;
2-(methylamino)-2-oxoethyl 4-(5-chloro-2-quinolyl)-1-piperazinecarboxylate;
2-amino-2-oxoethyl 4-isoquinol-3-yl-1-piperazinecarboxylate;
2-(methylamino)-2-oxoethyl 4-isoquinol-3-yl-1-piperazinecarboxylate;
2-(methylamino)-2-oxoethyl 4-(6-methoxy-3-isoquinolyl)-1-piperazinecarboxylate;
2-amino-2-oxoethyl 4-(3-chloro-6-isoquinolyl)-1-piperazinecarboxylate;
2-(methylamino)-2-oxoethyl 4-(3-chloro-6-isoquinolyl)-1-piperazinecarboxylate;
2-(methylamino)-2-oxoethyl 4-(5-bromo-2-pyridyl)-1,4-diazepane-1-carboxylate;
2-(methylamino)-2-oxoethyl 4-[5-(4-chlorophenyl)-2-pyridyl]-1,4-diazepane-1-carboxylate;
2-(methylamino)-2-oxoethyl 4-{5-[3-(trifluoromethyl)-phenyl]-2-pyridyl}-1,4-diazepane-1-carboxylate;
2-(methylamino)-2-oxoethyl 4-[5-(2-methoxyphenyl)-2-pyridyl]-1,4-diazepane-1-carboxylate;
2-(methylamino)-2-oxoethyl 4-[5-(3-methoxyphenyl)-2-pyridyl]-1,4-diazepane-1-carboxylate;
2-(methylamino)-2-oxoethyl 4-[5-(4-methoxyphenyl)-2-pyridyl]-1,4-diazepane-1-carboxylate;
2-(methylamino)-2-oxoethyl 4-{5-[4-(trifluoromethoxy)-phenyl]-2-pyridyl}-1,4-diazepane-1-carboxylate;
2-(methylamino)-2-oxoethyl 4-{5-[4-(trifluoromethyl)-phenyl]-2-pyridyl}-1,4-diazepane-1-carboxylate;
2-(methylamino)-2-oxoethyl 4-[5-(2,4-dichlorophenyl)-2-pyridyl]-1,4-diazepane-1-carboxylate;
2-(ethylamino)-2-oxoethyl 4-(3-phenyl-1,2,4-thiadiazol-5-yl)-1,4-diazepane-1-carboxylate.

7. Process for preparing a compound of formula (I) according to any one of Claims 1 to 6, comprising the step consisting in
reacting an amine of general formula (II), 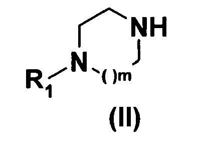 in which R₁ and m are as defined in the general formula (I) according to Claim 1,
with a carbonate of general formula (III), 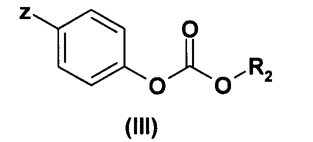 in which Z represents a hydrogen atom or a nitro group and R₂ is as defined in the general formula (I) according to Claim 1.

8. Process for preparing a compound of formula (I) according to any one of Claims 1 to 6, comprising the step consisting in converting the carbamate-ester of general formula (Ia) 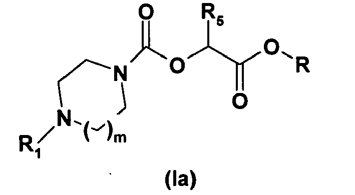 in which m, R₁ and R₅ are as defined in the general formula (I) according to Claim 1 and R represents a methyl or ethyl group,
via aminolysis using an amine of general formula R₆NH₂ in which R₆ is as defined in the general formula (I) according to Claim 1.

9. Compound corresponding to the general formula (III), which is 2-(methylamino)-2-oxoethyl 4-nitrophenyl carbonate.

10. Compound corresponding to the general formula (Ia), 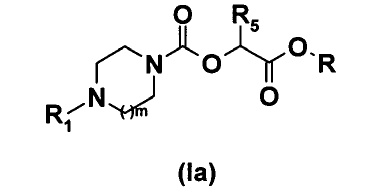 in which m, R₁ and R₅ are as defined in the general formula (I) according to Claim 1 and R represents a methyl or ethyl group.

11. Compound of formula (I) according to any one of Claims 1 to 6, in the form of base, of pharmaceutically acceptable acid-addition salt, of hydrate or of solvate, for its use as a medicament.

12. Pharmaceutical composition containing at least one compound of formula (I) according to any one of Claims 1 to 6, in the form of base, of pharmaceutically acceptable acid-addition salt, of hydrate or of solvate and optionally one or more pharmaceutically acceptable excipients.

13. Use of a compound of formula (I) according to any one of Claims 1 to 6, in the form of base, of pharmaceutically acceptable acid-addition salt, of hydrate or of solvate, for the preparation of a medicament for preventing or treating a pathology in which the endogenous cannabinoids and/or any other substrate metabolized by the enzyme FAAH are involved.

14. Use of a compound of formula (I) according to any one of Claims 1 to 6, in the form of base, of pharmaceutically acceptable salt, of hydrate or of solvate, for the preparation of a medicament for preventing or treating acute or chronic pain, vertigo, vomiting, nausea, eating behaviour disorders, neurological and psychiatric pathologies, acute or chronic neurodegenerative diseases, epilepsy, sleeping disorders, cardiovascular diseases, renal ischaemia, cancers, disorders of the immune system, allergic diseases, parasitic, viral or bacterial infectious diseases, inflammatory diseases, osteoporosis, ocular complaints, pulmonary complaints, gastrointestinal diseases or urinary incontinence.

15. Use according to Claim 14, **characterized in that** the acute or chronic pain is of neurogenic or peripheral type or associated with inflammatory diseases.

16. Use according to Claim 14, **characterized in that** the eating behaviour disorders correspond to anorexia and cachexia.

17. Use according to Claim 14, **characterized in that** the neurological and psychiatric pathologies are chosen from the following pathologies: tremor, dyskinaesia, dystonia, spasticity, compulsive and obsessive behaviour, Tourette's syndrome, depression, anxiety, mood disorders and psychoses.

18. Use according to Claim 14, **characterized in that** the acute and chronic neurogenic diseases are chosen from the following diseases: Parkinson's disease, Alzheimer's disease, senile dementia, Huntington's chorea, and lesions related to cerebral ischaemia and to cranial and medullary trauma.

19. Use according to Claim 14, **characterized in that** the cardiovascular diseases are chosen from the following diseases: hypertension, cardiac arrhythmia, arteriosclerosis, heart attack and cardiac ischaemia.

20. Use according to Claim 14, **characterized in that** the cancers are chosen from benign skin tumours, cerebral papillomas and tumours, and prostate tumours.

21. Use according to Claim 14, **characterized in that** the immune system disorders are autoimmune diseases.

22. Use according to Claim 14, **characterized in that** the allergic diseases are chosen from: immediate or delayed hypersensitivity, allergic rhinitis or allergic conjunctivitis, and contact dermatitis.

23. Use according to Claim 14, **characterized in that** the inflammatory diseases are articular diseases.

24. Use according to Claim 14, **characterized in that** the pulmonary conditions are chosen from: respiratory pathway diseases, bronchospasms, coughing, asthma, chronic bronchitis, chronic obstruction of the respiratory pathways, and emphysema.

25. Use according to Claim 14, **characterized in that** the gastrointestinal diseases are chosen from: irritable bowel syndrome, intestinal inflammatory disorders, ulcers and diarrhoea.

## Patentansprüche

1. Verbindung nach der Formel (I) 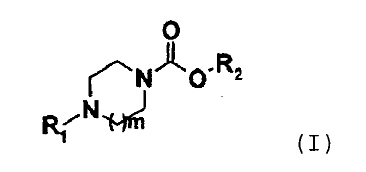 in welcher
m für eine ganze Zahl steht, die gleich 1 oder 2 ist;
R₁ für eine Gruppe steht, die insbesondere aus den Gruppen Phenyl, Pyridinyl, Pyridazinyl, Pyrimidinyl, Pyrazinyl, Triazinyl, Oxazolyl, Thiazolyl, Imidazolyl, Oxadiazolyl, Thiadiazolyl, Triazolyl, Naphthyl, Chinolinyl, Tetrahydrochinolinyl, Isochinolinyl, Tetrahydroisochinolinyl, Phthalazinyl, Chinazolinyl, Chinoxalinyl, Cinnolinyl, Naphthyridinyl, Benzofuranyl, Dihydrobenzofuranyl, Benzothienyl, Dihydrobenzothienyl, Indolyl, Benzoxazolyl, Benzisoxazolyl, Benzothiazolyl, Benzisothiazolyl, Benzimidazolyl, Indazolyl, Pyrrolopyridinyl, Furopyridinyl, Dihydrofuropyridinyl, Thienopyridinyl, Dihydrothienopyridinyl, Imidazopyridinyl, Imidazopyrimidinyl, Pyrazolopyridinyl, Oxazolopyridinyl, Isoxazolopyridinyl, Thiazolopyridinyl und Isothiazolopyridinyl gewählt ist,
wobei diese Gruppe möglicherweise mit einer oder mehreren Gruppen R₃, die identisch oder verschiedenartig voneinander sein können, oder mit einer Gruppe R₄ substituiert ist;
R₂ für eine Gruppe nach der allgemeinen Formel CHR₅CONHR₆ steht,
R₃ für ein Halogenatom oder eine Gruppe des Typs Hydroxy, Cyano, Nitro, C₁₋₆-Alkyl, C₁₋₆-Alkoxy, C₁₋₆-Thioalkyl, C₁₋₆-Fluoralkyl, C₁₋₆-Fluoralkoxy, -O- (C₂₋₃-Alkylen) -, -O- (C₁₋₃-Alkylen)-O-, C₁₋₆-Fluorthioalkyl, C₃₋₇-Cycloalkyl, C₃₋₇-Cycloalkyl-C₁₋₃-Alkylen, Piperidinyl, Benzyloxy, Piperazinyl, Pyrrolidinyl, Morpholinyl, Phenyloxy, NR₇R₈, NHCOR₇, NHSO₂R₇, COR₇, CO₂R₇, CONR₇R₈, SO₂R₇ oder SO₂NR₇R₈ steht,
R₄ für eine Gruppe steht, die insbesondere aus den Gruppen Phenyl, Benzofuranyl, Naphthyl, Pyridinyl, Pyrimidinyl, Pyridazinyl, Pyrazinyl, Triazinyl, Oxazolyl, Thiazolyl, Imidazolyl, Oxadiazolyl, Thiadiazolyl, Triazolyl, Chinolinyl, Tetrahydrochinolinyl, Isochinolinyl, Tetrahydroisochinolinyl, Phthalazinyl, Chinazolinyl, Chinoxalinyl, Naphthyridinyl, Cinnolinyl, Imidazolpyrimidinyl, Benzothienyl, Indolyl, Benzoxazolyl, Benzisoxazolyl, Benzothiazolyl, Benzisothiazolyl, Benzimidazolyl, Indazolyl, Pyrrolopyridinyl, Furopyridinyl, Dihydrofuropyridinyl, Thienopyridinyl, Dihydrothienopyridinyl, Imidazopyridinyl, Imidazopyrimidinyl, Pyrazopyridinyl, Oxazolopyridinyl, Isoxazolopyridinyl, Thiazolopyridinyl und Isothiazolopyridinyl gewählt ist,
wobei die Gruppe(n) R₄ möglicherweise mit einer oder mehreren Gruppen R₃, die identisch oder verschiedenartig voneinander sein können, substituiert ist/sind;
R₅ für ein Wasserstoffatom oder eine C₁₋₃-Alkylgruppe steht;
R₆ für ein Wasserstoffatom oder eine Gruppe des Typs C₁₋₆-Alkyl, C₃₋₇-Cycloalkyl oder C₃₋₇-Cycloalkyl-C₁₋₃-alkylen steht;
R₇ und R₈, unabhängig voneinander, für ein Wasserstoffatom, eine C₁₋₃-Alkylgruppe oder eine Phenylgruppe stehen;
und in Form einer Base, eines Salzes, welches bei Säurezusatz gebildet wird, eines Hydrates oder eines Solvates.

2. Verbindung nach Formel (I) nach Anspruch 1, **dadurch gekennzeichnet, dass**
m für eine ganze Zahl steht, die gleich 1 oder 2 ist;
R₁ für eine Gruppe steht, die insbesondere aus den Gruppen Phenyl, Pyridinyl, Pyrimidinyl, Pyrazinyl, Naphthyl, Chinolinyl, Isochinolinyl, Benzisoxazolyl und Thienopyridinyl gewählt ist, wobei diese Gruppe möglicherweise mit einer oder mehreren Gruppen R₃, die identisch oder verschiedenartig voneinander sein können, substituiert ist;
R₂ für eine Gruppe nach der allgemeinen Formel CHR₅CONHR₆ steht;
R₃ für ein Halogenatom oder eine Gruppe des Typs Cyano, C₁₋₆-Alkyl, C₁₋₆-Alkoxy, C₁₋₆-Fluoralkyl, C₁₋₆-Fluoralkoxy, -O-(C₂₋₃-Alkylen) - oder Phenyloxy steht;
R₅ für ein Wasserstoffatom steht;
R₆ für ein Wasserstoffatom oder eine C₁₋₆-Alkylgruppe steht.

3. Verbindung nach Formel (I) nach den Ansprüchen 1 oder 2, **dadurch gekennzeichnet, dass**
m gleich 1 ist;
R₁ für eine Gruppe steht, die insbesondere aus den Gruppen Pyridinyl, Pyrimidinyl, Pyrazinyl, Chinolinyl und Isochinolinyl gewählt ist, wobei diese Gruppe möglicherweise mit einer Gruppe R₃ substituiert ist;
R₂ für eine Gruppe nach der allgemeinen Formel CHR₅CONHR₆ steht;
R₃ für ein Halogenatom oder eine Gruppe des Typs C₁₋₆-Alkyl, C₁₋₆-Alkoxy oder C₁₋₆-Fluoralkyl steht;
R₅ für ein Wasserstoffatom steht;
R₆ für ein Wasserstoffatom oder eine C₁₋₆-Alkylgruppe steht.

4. Verbindung nach Formel (I) nach Anspruch 1, **dadurch gekennzeichnet, dass**
m für eine ganze Zahl steht, die gleich 1 oder 2 ist;
R₁ für eine Gruppe steht, die insbesondere aus den Gruppen Phenyl, Pyridinyl, Pyridazinyl, Pyrimidinyl und Thiadiazolyl gewählt ist, wobei diese Gruppe möglicherweise mit einer Gruppe R₄ substituiert ist;
R₄ für eine Gruppe steht, die insbesondere aus den Gruppen Phenyl, Benzofuranyl und Naphthyl gewählt ist; wobei die Gruppe R₄ mit einer oder mehreren Gruppen R₃, die identisch oder verschiedenartig voneinander sein können, substituiert sein kann;
R₂ für eine Gruppe nach der allgemeinen Formel CHR₅CONHR₆ steht;
R₃ für ein Halogenatom oder eine Gruppe des Typs Nitro, C₁₋₆-Alkyl, C₁₋₆-Alkoxy, C₁₋₆-Fluoralkyl, C₁₋₆-Fluoralkoxy, -O-(C₁₋₃-Alkylen)-O- oder Benzyloxy steht;
R₅ für ein Wasserstoffatom steht;
R₆ für ein Wasserstoffatom oder eine Gruppe des Typs C₁₋₆-Alkyl oder C₃₋₇-Cycloalkyl-C₁₋₃-alkylen steht.

5. Verbindung nach Formel (I) nach den Ansprüchen 1 oder 4, **dadurch gekennzeichnet, dass**
m gleich 1 ist;
R₁ für eine Gruppe steht, die insbesondere aus den Gruppen Phenyl, Pyridinyl, Pyridazinyl und Pyrimidinyl gewählt ist,
wobei diese Gruppe möglicherweise mit einer Gruppe R₄ substituiert ist;
R₄ für eine Gruppe steht, die insbesondere aus den Gruppen Phenyl, Benzofuranyl und Naphthyl gewählt ist; wobei die Gruppe R₄ mit einer oder mehreren Gruppen R₃, die identisch oder verschiedenartig voneinander sein können, substituiert sein kann;
R₂ für eine Gruppe nach der allgemeinen Formel CHR₅CONHR₆ steht;
R₃ für ein Halogenatom oder eine Gruppe des Typs Nitro, C₁₋₆-Alkyl, C₁₋₆-Alkoxy, C₁₋₆-Fluoralkyl, C₁₋₆-Fluoralkoxy, -O-(C₁₋₃-Alkylen)-O- oder Benzyloxy steht;
R₅ für ein Wasserstoffatom steht;
R₆ für ein Wasserstoffatom oder eine C₁₋₆-Alkylgruppe steht.

6. Verbindung nach Formel (I) nach Anspruch 1, welche aus des folgenden Verbindungen gewählt ist:
2-(Methylamino)-2-oxoethyl-4-phenylpiperazin-1-carboxylat;
2-(Methylamino)-2-oxoethyl-4-(2-chlorphenyl)piperazin-1-carboxylat;
2-(Methylamino)-2-oxoethyl-4-(3-chlorphenyl)piperazin-1-carboxylat;
2-(Methylamino)-2-oxoethyl-4-(4-chlorphenyl)piperazin-1-carboxylat;
2-(Methylamino)-2-oxoethyl-4-(2-fluorphenyl)piperazin-1-carboxylat;
2-(Methylamino)-2-oxoethyl-4-(3-fluorphenyl)piperazin-1-carboxylat;
2-(Methylamino)-2-oxoethyl-4-(4-fluorphenyl)piperazin-1-carboxylat;
2-(Methylamino)-2-oxoethyl-4-(4-cyanophenyl)piperazin-1-carboxylat;
2-(Methylamino)-2-oxoethyl-4-(2-methylphenyl)piperazin-1-carboxylat;
2-(Methylamino)-2-oxoethyl-4-(3-methylphenyl)piperazin-1-carboxylat;
2-(Methylamino)-2-oxoethyl-4-(4-methylphenyl)piperazin-1-carboxylat;
2-(Methylamino)-2-oxoethyl-4-(2-methoxyphenyl)piperazin-1-carboxylat;
2-(Methylamino)-2-oxoethyl-4-(3-methoxyphenyl)piperazin-1-carboxylat;
2-(Methylamino)-2-oxoethyl-4-(4-methoxyphenyl)piperazin-1-carboxylat;
2-(Methylamino)-2-oxoethyl-4-(6-fluor-3,4-dihydro-2H-chromen-8-yl)piperazin-1-carboxylat;
2-(Methylamino)-2-oxoethyl-4-[2-(2,2,2-trifluorethoxy)phenyl]piperazin-1-carboxylat;
2-(Methylamino)-2-oxoethyl-4-[2-(trifluormethyl)phenyl]piperazin-1-carboxylat;
2-(Methylamino)-2-oxoethyl-4-[3-(trifluormethyl)phenyl]piperazin-1-carboxylat;
2-Amino-2-oxoethyl-4-[3-trifluormethyl)phenyl]piperazin-1-carboxylat;
2-(Methylamino)-2-oxoethyl-4-[4-(trifluormethyl)phenyl]piperazin-1-carboxylat;
2-(Methylamino)-2-oxoethyl-4-(2,3-dimethylphenyl)piperazin-1-carboxylat;
2-(Methylamino)-2-oxoethyl-4-(2,4-dimethylphenyl)piperazin-1-carboxylat;
2-(Methylamino)-2-oxoethyl-4-(2,5-dimethylphenyl)piperazin-1-carboxylat;
2-(Methylamino)-2-oxoethyl-4-(3,4-dimethylphenyl)piperazin-1-carboxylat;
2-(Methylamino)-2-oxoethyl-4-(3,4-dichlorphenyl)piperazin-1-carboxylat;
2-(Methylamino)-2-oxoethyl-4-(3-chlor-4-fluorphenyl)piperazin-1-carboxylat;
2-(Methylamino)-2-oxoethyl-4-[4-chlor-3-(trifluormethyl)phenyl]piperazin-1-carboxylat;
2-(Methylamino)-2-oxoethyl-4-(5-chlor-2-methoxyphenyl)piperazin-1-carboxylat;
2-(Methylamino)-2-oxoethyl-4-naphthalin-1-ylpiperazin-1-carboxylat;
2-(Methylamino)-2-oxoethyl-4-biphenyl-2-ylpiperazin-1-carboxylat;
2-(Methylamino)-2-oxoethyl-4-(2-phenoxyphenyl) piperazin-1-carboxylat;
2-(Methylamino)-2-oxoethyl-4-biphenyl-3-ylpiperazin-1-carboxylat;
2-(Methylamino)-2-oxoethyl-4-biphenyl-4-ylpiperazin-1-carboxylat;
2-(Methylamino)-2-oxoethyl-4-(4'-chlorbiphenyl-4-yl)piperazin-1-carboxylat;
2-(Methylamino)-2-oxoethyl-4-(4'-fluorbiphenyl-4-yl)piperazin-1-carboxylat;
2-(Methylamino)-2-oxoethyl-4-(4'-nitrobiphenyl-4-yl)piperazin-1-carboxylat;
2-(Methylamino)-2-oxoethyl-4-[3'-(trifluormethyl)biphenyl-4-yl]piperazin-1-carboxylat;
2-(Methylamino)-2-oxoethyl-4-[4'-(trifluormethyl)biphenyl-4-yl]piperazin-1-carboxylat;
2-(Methylamino)-2-oxoethyl-4-[3'-(1-methylethyl)biphenyl-4-yl]piperazin-1-carboxylat;
2-(Methylamino)-2-oxoethyl-4-[4'-(1-methylethyl)biphenyl-4-yl]piperazin-1-carboxylat;
2-(Methylamino)-2-oxoethyl-4-(3'-methoxybiphenyl-4-yl)piperazin-1-carboxylat;
2-(Methylamino)-2-oxoethyl-4-(3'-ethoxybiphenyl-4-yl)piperazin-1-carboxylat;
2-(Methylamino)-2-oxoethyl-4-(4'-ethoxybiphenyl-4-yl)piperazin-1-carboxylat;
2-(Methylamino)-2-oxoethyl-4-[4'-(trifluormethoxy)biphenyl-4-yl]piperazin-1-carboxylat;
2-Amino-2-oxoethyl-4-[4'-(trifluormethoxy)biphenyl-4-yl]piperazin-1-carboxylat;
2-(Methylamino)-2-oxoethyl-4-[2'-(benzyloxy)biphenyl-4-yl]piperazin-1-carboxylat;
2-(Methylamino)-2-oxoethyl-4-(2',4'-dichlorbiphenyl-4-yl)piperazin-1-carboxylat;
2-(Methylamino)-2-oxoethyl-4-(2'-fluor-3'-methoxybiphenyl-4-yl)piperazin-1-carboxylat;
2-(Methylamino)-2-oxoethyl-4-(3'-fluor-4'-methoxybiphenyl-4-yl)piperazin-1-carboxylat;
2-(Methylamino)-2-oxoethyl-4-[4'-(benzyloxy)-3'-fluorbiphenyl-4-yl]piperazin-1-carboxylat;
2-(Methylamino)-2-oxoethyl-4-(2',5'-dimethoxybiphenyl-4-yl)piperazin-1-carboxylat;
2-(Methylamino)-2-oxoethyl-4-(5'-chlor-2'-methoxybiphenyl-1-yl)piperazin-1-carboxylat;
2-(Methylamino)-2-oxoethyl-4-[4-(1,3-benzodioxol-5-yl)phenyl]piperazin-1-carboxylat;
2-(Methylamino)-2-oxoethyl-4-[4-(1-benzofuran-2-yl)phenyl]piperazin-1-carboxylat;
2-(Methylamino)-2-oxoethyl-4-(4-naphthalin-1-ylphenyl)piperazin-1-carboxylat;
2-(Methylamino)-2-oxoethyl-4-[4-(4-methylnaphthalin-1-yl)phenyl]piperazin-1-carboxylat;
2-(Methylamino)-2-oxoethyl-4-(4-naphthalin-2-ylphenyl)piperazin-1-carboxylat;
2-(Methylamino)-2-oxoethyl-4-pyridin-2-ylpiperazin-1-carboxylat;
2-(Methylamino)-2-oxoethyl-4-pyridin-4-ylpiperazin-1-carboxylat;
2-(Methylamino)-2-oxoethyl-4-(5-methylpyridin-2-yl)piperazin-1-carboxylat;
2-(Ethylamino)-2-oxoethyl-4-(5-methylpyridin-2-yl)piperazin-1-carboxylat;
2-[(Cyclopropylmethyl)amino]-2-oxoethyl-4-(5-methylpyridin-2-yl)piperazin-1-carboxylat;
2-(Methylamino)-2-oxoethyl-4-[5-(2-methylpropyl)pyridin-2-yl]piperazin-1-carboxylat;
2-(Methylamino)-2-oxoethyl-4-(6-brompyridin-3-yl)piperazin-1-carboxylat;
2-(Methylamino)-2-oxoethyl-4-(6-propylpyridin-3-yl)piperazin-1-carboxylat;
2-(Methylamino)-2-oxoethyl-4-[6-(2-methylpropyl)pyridin-3-yl]piperazin-1-carboxylat;
2-(Methylamino)-2-oxoethyl-4-pyrazin-2-ylpiperazin-1-carboxylat;
2-(Ethylamino)-2-oxoethyl-4-pyrazin-2-ylpiperazin-1-carboxylat;
2-(Methylamino)-2-oxoethyl-4-(5-ethylpyrimidin-2-yl)piperazin-1-carboxylat;
2-[(Cyclopropylmethyl)amino]-2-oxoethyl-4-(5-ethylpyrimidin-2-yl)piperazin-1-carboxylat;
2-(Methylamino)-2-oxoethyl-4-[4-(trifluormethyl)pyrimidin-2-yl]piperazin-1-carboxylat;
2-(Ethylamino)-2-oxoethyl-4-[4-(trifluormethyl)pyrimidin-2-yl]piperazin-1-carboxylat;
2-(Methylamino)-2-oxoethyl-4-(6-methoxy-1,2-benzisoxazol-3-yl)piperazin-1-carboxylat;
2-(Methylamino)-2-oxoethyl-4-thieno[3,2-c]pyridin-4-ylpiperazin-1-carboxylat;
2-(Methylamino)-2-oxoethyl-4-[5-(4-chlorphenyl)pyridin-2-yl]piperazin-1-carboxylat;
2-(Methylamino)-2-oxoethyl-4-{5-[3-(trifluormethyl)phenyl]pyridin-2-yl}piperazin-1-carboxylat;
2-Amino-2-oxoethyl-4-{5-[3-(trifluormethyl)phenyl]pyridin-2-yl}piperazin-1-carboxylat;
2-Amino-2-oxoethyl-4-{5-[4-(trifluormethyl)phenyl]pyridin-2-yl}piperazin-1-carboxylat;
2-(Methylamino)-2-oxoethyl-4-{5-[4-(trifluormethyl)phenyl]pyridin-2-yl}piperazin-1-carboxylat;
2-(Ethylamino)-2-oxoethyl-4-{5-[4-(trifluormethyl)phenyl]pyridin-2-yl}piperazin-1-carboxylat;
2-[(Cyclopropylmethyl)amino]-2-oxoethyl-4-{5-[4-(trifluormethyl)phenyl]pyridin-2-yl}piperazin-1-carboxylat;
1-Methyl-2-(methylamino)-2-oxoethyl-4-{5-[4-(trifluormethyl)phenyl]pyridin-2-yl}piperazin-1-carboxylat;
2-(Methylamino)-2-oxoethyl-4-{5-[4-(trifluormethoxy)phenyl]pyridin-2-yl}piperazin-1-carboxylat;
2-(Methylamino)-2-oxoethyl-4-[5-(2,4-dichlorphenyl)pyridin-2-yl]piperazin-1-carboxylat;
2-(Methylamino)-2-oxoethyl-4-{6-[3-(trifluormethyl)phenyl]pyridin-3-yl}piperazin-1-carboxylat;
2-Amino-2-oxoethyl-4-{6-[3-(trifluormethyl)phenyl]pyridin-3-yl}piperazin-1-carboxylat;
2-(Methylamino)-2-oxoethyl-4-{6-[3-(trifluormethyl)phenyl]pyridazin-3-yl}piperazin-1-carboxylat;
2-(Methylamino)-2-oxoethyl-4-[5-(3-chlorphenyl)pyrimidin-2-yl]piperazin-1-carboxylat;
2-(Methylamino)-2-oxoethyl-4-chinolin-2-ylpiperazin-1-carboxylat;
2-Amino-2-oxoethyl-4-(5-chlorchinolin-2-yl)piperazin-1-carboxylat;
2-(Methylamino)-2-oxoethyl-4-(5-chlorchinolin-2-yl)piperazin-1-carboxylat;
2-Amino-2-oxoethyl-4-isochinolin-3-ylpiperazin-1-carboxylat;
2-(Methylamino)-2-oxoethyl-4-isochinolin-3-ylpiperazin-1-carboxylat;
2-(Methylamino)-2-oxoethyl-4-(6-methoxyisochinolin-3-yl)piperazin-1-carboxylat;
2-Amino-2-oxoethyl-4-(3-chlorisochinolin-6-yl)piperazin-1-carboxylat;
2-(Methylamino)-2-oxoethyl-4-(3-chlorisochinolin-6-yl)piperazin-1-carboxylat;
2-(Methylamino)-2-oxoethyl-4-(5-brompyridin-2-yl)-1,4-diazepan-1-carboxylat;
2-(Methylamino)-2-oxoethyl-4-[5-(4-chlorphenyl)pyridin-2-yl]-1,4-diazepan-1-carboxylat;
2-(Methylamino)-2-oxoethyl-4-{5-[3-(trifluormethyl)phenyl]pyridin-2-yl}-1,4-diazepan-1-carboxylat;
2-(Methylamino)-2-oxoethyl-4-[5-(2-methoxyphenyl)pyridin-2-yl]-1,4-diazepan-1-carboxylat;
2-(Methylamino)-2-oxoethyl-4-[5-(3-methoxyphenyl) pyridin-2-yl]-1,4-diazepan-1-carboxylat;
2-(Methylamino)-2-oxoethyl-4-[5-(4-methoxyphenyl)pyridin-2-yl]-1,4-diazepan-1-carboxylat;
2-(Methylamino)-2-oxoethyl-4-{5-[4-(trifluormethoxy)phenyl]pyridin-2-yl}-1,4-diazepan-1-carboxylat;
2-(Methylamino)-2-oxoethyl-4-{5-[4-(trifluormethyl)phenyl]pyridin-2-yl}-1,4-diazepan-1-carboxylat;
2-(Methylamino)-2-oxoethyl-4-[5-(2,4-dichlorphenyl)pyridin-2-yl]-1,4-diazepan-1-carboxylat;
2-(Ethylamino)-2-oxoethyl-4-(3-phenyl-1,2,4-thiadiazol-5-yl)-1,4-diazepan-1-carboxylat.

7. Herstellungsverfahren für eine Verbindung nach Formel (I) nach einem beliebigen der Ansprüche 1 bis 6, welches einen Schritt umfasst, der darin besteht,
ein Amin nach der allgemeinen Formel (II), 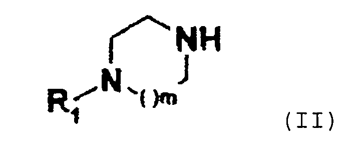 in welcher R₁ und m der Definition für die allgemeine Formel (I) nach Anspruch 1 entsprechen,
mit einem Carbonat umzusetzen, welches der allgemeinen Formel (III) 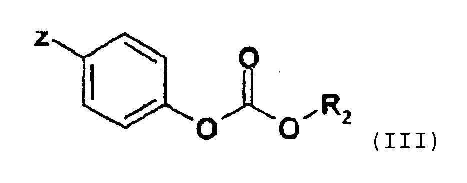 entspricht, wobei Z für ein Wasserstoffatom oder eine Nitrogruppe steht und R₂ der Definition für die allgemeine Formel (I) nach Anspruch 1 entspricht.

8. Herstellungsverfahren für eine Verbindung nach Formel (I) nach einem beliebigen der Ansprüche 1 bis 6, welches einen Schritt umfasst, der darin besteht, den Carbamatester nach der allgemeinen Formel (Ia) 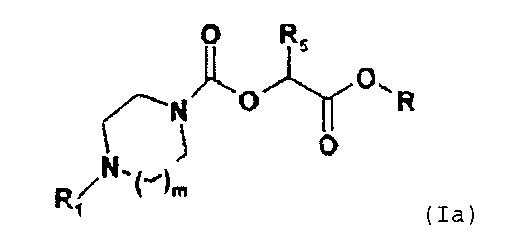 in welcher m, R₁ und R₅ der Definition für die allgemeine Formel (I) nach Anspruch 1 entsprechen und R für eine Gruppe des Typs Methyl oder Ethyl steht,
durch Aminolyse umzuwandeln, und zwar mittels eines Amins nach der allgemeinen Formel R₆NH₂, in welcher R₆ der Definition für die allgemeine Formel (I) nach Anspruch 1 entspricht.

9. Verbindung, welche der allgemeinen Formel (III) entspricht, wobei es sich bei der Verbindung um 2-(Methylamino)-2-oxoethyl-4-nitrophenylcarbonat handelt.

10. Verbindung nach der allgemeinen Formel (Ia), 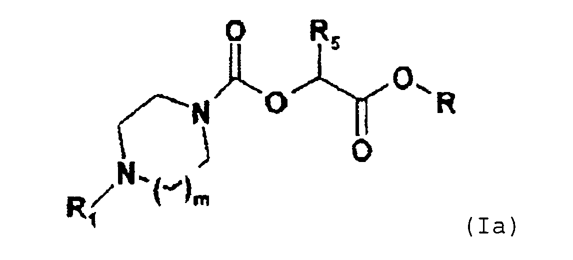 in welcher m, R₁ und R₅ der Begriffsbestimmungen für die allgemeine Formel (I) nach Anspruch 1 entsprechen und R für eine Gruppe des Typs Methyl oder Ethyl steht.

11. Verbindung nach Formel (I) nach einem beliebigen der Ansprüche 1 bis 6, welche in Form einer Base, eines Salzes, das bei Säurezusatz gebildet wird, eines Hydrates oder eines pharmazeutisch unbedenklichen Solvates vorliegt, und welche als Arzneimittel verwendet wird.

12. Pharmazeutische Zusammensetzung, welche mindestens eine Verbindung nach Formel (I) nach einem beliebigen der Ansprüche 1 bis 6, die in Form einer Base, eines Salzes, das bei Säurezusatz gebildet wird, eines Hydrates oder eines pharmazeutisch unbedenklichen Solvates vorliegt, sowie möglicherweise einen oder mehrere pharmazeutisch unbedenkliche Trägerstoff(e) enthält.

13. Verwendung einer Verbindung nach Formel (I) nach einem beliebigen der Ansprüche 1 bis 6, welche in Form einer Base, eines Salzes, das bei Säurezusatz gebildet wird, eines Hydrates oder eines pharmazeutisch unbedenklichen Solvates vorliegt, zur Herstellung eines Arzneimittels, das dazu bestimmt ist, ein Krankheitsbild zu behandeln oder diesem vorzubeugen, bei welchem endogene Cannabinoide und/oder beliebige andere Substrate, die von dem Enzym FAAH verstoffwechselt werden, eine Rolle spielen.

14. Verwendung einer Verbindung nach Formel (I) nach einem beliebigen der Ansprüche 1 bis 6, welche in Form einer Base, eines Salzes, das bei Säurezusatz gebildet wird, eines Hydrates oder eines pharmazeutisch unbedenklichen Solvates vorliegt, zur Herstellung eines Arzneimittels, das dazu bestimmt ist, akute oder chronische Schmerzen, Schwindelgefühl, Erbrechen, Übelkeit, Ernährungsstörungen, neurologische oder psychiatrische Erkrankungen, akute oder chronische neurodegenerative Krankheiten, Epilepsie, Schlafstörungen, Herz-Kreislauf-Krankheiten, renale Ischämie, Krebs, Störungen des Immunsystems, allergische Krankheiten, Infektionskrankheiten, die durch Parasiten, Viren oder Bakterien ausgelöst werden, krankhafte Entzündungen, Osteoporose, Augenerkrankungen, Lungenerkrankungen, Krankheiten des Magen-DarmTraktes oder Harninkontinenz zu behandeln oder diesen vorzubeugen.

15. Verwendung nach Anspruch 14, **dadurch gekennzeichnet, dass** es sich bei den akuten oder chronische Schmerzen um solche handelt, die neurogen oder peripher sind oder die mit krankhaften Entzündungen in Verbindung stehen.

16. Verwendung nach Anspruch 14, **dadurch gekennzeichnet, dass** die Ernährungsstörungen Formen der Anorexie und der Kachexie entsprechen.

17. Verwendung nach Anspruch 14, **dadurch gekennzeichnet, dass** die neurologischen und psychiatrischen Erkrankungen aus den folgenden Erkrankungen gewählt sind: Zittern, Störungen des Bewegungsablaufes, Dystonien, spastische Störungen, Zwangsstörungen, Tourette-Syndrom, Depression, Angstzustände, krankhafte Stimmungsschwankungen, Psychosen.

18. Verwendung nach Anspruch 14, **dadurch gekennzeichnet, dass** die akuten und chronischen neurodegenerativen Krankheiten aus den folgenden Krankheiten gewählt sind: Parkinson-Krankheit, Alzheimer-Krankheit, Altersdemenz, Chorea Huntington und Verletzungen, die mit einer zerebralen Ischämie und mit Traumata des Schädels und der Medulla in Verbindung stehen.

19. Verwendung nach Anspruch 14, **dadurch gekennzeichnet, dass** die Herz-Kreislauf-Krankheiten aus den folgenden Krankheiten gewählt sind: Bluthochdruck, Herzrhythmusstörungen, Arteriosklerose, Herzinfarkt, Ischämien des Herzens.

20. Verwendung nach Anspruch 14, **dadurch gekennzeichnet, dass** die Krebserkrankungen aus gutartigen Tumoren der Haut, Papillomen und Hirntumoren sowie Prostatatumoren gewählt sind.

21. Verwendung nach Anspruch 14, **dadurch gekennzeichnet, dass** die Störungen des Immunsystems aus den Autoimmunkrankheiten gewählt sind.

22. Verwendung nach Anspruch 14, **dadurch gekennzeichnet, dass** die allergischen Krankheiten aus der sofortigen oder verzögerten Hypersensibilität, den allergischen Erkrankungen der Nasenschleimhäute und der Bindehaut des Auges sowie den Hauterkrankungen nach Kontakt gewählt sind.

23. Verwendung nach Anspruch 14, **dadurch gekennzeichnet, dass** es sich bei den krankhaften Entzündungen um Krankheiten der Gelenke handelt.

24. Verwendung nach Anspruch 14, **dadurch gekennzeichnet, dass** die Lungenerkrankungen aus den Krankheiten der Atemwege, Bronchospasmen, Husten, Asthma, chronischer Bronchitis, chronischer Verstopfung der Atemwege und Emphysem gewählt sind.

25. Verwendung nach Anspruch 14, **dadurch gekennzeichnet, dass** die Krankheiten des Magen-DarmTraktes aus den folgenden gewählt sind: Reizdarm-Syndrom, entzündliche Darmstörungen, Geschwüre und Durchfallerscheinungen.
